# EUROPEAN PATENT SPECIFICATION

(11) **EP 3 261 723 B1**
(45) Date of publication and mention of the grant of the patent: **07.04.2021**
(21) Application number: 16704579.8
(22) Date of filing: 09.02.2016
(51) Int. Cl.: A61K 35/747, A61K 31/665, A23L 33/135, A61P 41/00

(54) **PROBIOTIC LACTOBACILLUS PLANTARUM STRAINS FOR URINARY TRACT INFECTIONS**
PROBIOTISCHE LACTOBACILLUS PLANTARUM STÄMME FÜR HARNWEGSINFEKTIONEN
SOUCHES PROBIOTIQUES DE LACTOBACILLUS PLANTARUM POUR LE TRAITEMENT DES INFECTIONS DES VOIES URINAIRES

(30) Priority: 11.02.2015 EP 15382051
(43) Date of publication of application: 03.01.2018
(73) Proprietor: AB-Biotics S.A., 08193 Cerdanyola del Valles (Barcelona) (ES)
(72) Inventor: SANTAS GUTIÉRREZ, Jonathan, 08860 Castelldefels (ES); CUÑÉ CASTELLANA, Jordi, 08191 Rubí (ES); LÁZARO MALLÉN, Elisabet, 08030 Barcelona (ES)
(74) Representative: ZBM Patents ApS
(86) International application number: PCT/EP2016/052747
(87) International publication number: WO 2016/128414

(56) References cited:
- EP-A1- 2 000 530
- EP-B1- 1 137 423
- WO-A1-2006/045347
- WO-A1-2013/034975
- WO-A1-2014/184643
- PER-GÃRAN LARSSON ET AL: "Extended antimicrobial treatment of bacterial vaginosis combined with human lactobacilli to find the best treatment and minimize the risk of relapses", BMC INFECTIOUS DISEASES, BIOMED CENTRAL, LONDON, GB, vol. 11, no. 1, 19 August 2011 (2011-08-19), page 223, XP021108821, ISSN: 1471-2334, DOI: 10.1186/1471-2334-11-223
- ANUKAM K ET AL: "Augmentation of antimicrobial metronidazole therapy of bacterial vaginosis with oral probiotic Lactobacillus rhamnosus GR-1 and Lactobacillus reuteri RC-14: randomized, double-blind, placebo controlled trial", MICROBES AND INFECTION, ELSEVIER, PARIS, FR, vol. 8, no. 6, 1 May 2006 (2006-05-01), pages 1450-1454, XP028072376, ISSN: 1286-4579, DOI: 10.1016/J.MICINF.2006.01.003 [retrieved on 2006-05-01]
- BARRONS R ET AL: "Use of Lactobacillus probiotics for bacterial genitourinary infections in women: A review", CLINICAL THERAPEUTICS, EXCERPTA MEDICA, PRINCETON, NJ, US, vol. 30, no. 3, 1 March 2008 (2008-03-01), pages 453-468, XP022593705, ISSN: 0149-2918, DOI: 10.1016/J.CLINTHERA.2008.03.013 [retrieved on 2008-03-01]
- TAKESHI MIKUNIYA ET AL: "Treatment of Pseudomonas aeruginosa biofilms with a combination of fluoroquinolones and fosfomycin in a rat urinary tract infection model", JOURNAL OF INFECTION AND CHEMOTHERAPY ; OFFICIAL JOURNAL OF THE JAPANESE SOCIETY OF CHEMOTHERAPY AND THE JAPANESE ASSOCIATION FORINFECTIOUS DISEASES, SPRINGER-VERLAG, TO, vol. 13, no. 5, 30 October 2007 (2007-10-30), pages 285-290, XP019546164, ISSN: 1437-7780, DOI: 10.1007/S10156-007-0534-7
- STRUS M ET AL: "Studies on the effects of probiotic Lactobacillus mixture given orally on vaginal and rectal colonization and on parameters of vaginal health in women with intermediate vaginal flora", EUROPEAN JOURNAL OF OBSTETRICS & GYNECOLOGY AND REPRODUCTIVEBIOLOGY, EXCERPTA MEDICA, AMSTERDAM, NL, vol. 163, no. 2, 1 August 2012 (2012-08-01), pages 210-215, XP002712127, ISSN: 0301-2115, DOI: 10.1016/J.EJOGRB.2012.05.001 [retrieved on 2012-06-19]
- DONDERS G: "Diagnosis and management of bacterial vaginosis and other types of abnormal vaginal bacterial flora: A review", OBSTETRICAL AND GYNECOLOGICAL SURVEY, WILLIAMS & WILKINS, BALTIMORE, MD, US, vol. 65, no. 7, 1 January 2010 (2010-01-01), pages 462-473, XP009180009, ISSN: 0029-7828
- HILLEBRAND LINDA ET AL: "Urinary tract infections in pregnant women with bacterial vaginosis", AMERICAN JOURNAL OF OBSTETRICS & GYNECOLOGY, vol. 186, no. 5, 24 September 2002 (2002-09-24), pages 916-917, XP029482831, ISSN: 0002-9378, DOI: 10.1067/MOB.2002.123987
- PER-GÃRAN LARSSON ET AL: "Extended antimicrobial treatment of bacterial vaginosis combined with human lactobacilli to find the best treatment and minimize the risk of relapses", BMC INFECTIOUS DISEASES, BIOMED CENTRAL, LONDON, GB, vol. 11, no. 1, 19 August 2011 (2011-08-19), page 223, XP021108821, ISSN: 1471-2334, DOI: 10.1186/1471-2334-11-223
- STRUS M ET AL: "Studies on the effects of probiotic Lactobacillus mixture given orally on vaginal and rectal colonization and on parameters of vaginal health in women with intermediate vaginal flora", EUROPEAN JOURNAL OF OBSTETRICS & GYNECOLOGY AND REPRODUCTIVE BIOLOGY, ELSEVIER IRELAND LTD, IE, vol. 163, no. 2, 1 August 2012 (2012-08-01), pages 210-215, XP002712127, ISSN: 0301-2115, DOI: 10.1016/J.EJOGRB.2012.05.001 [retrieved on 2012-06-19]
- SCHITO G C ET AL: "The ARESC study: an international survey on the antimicrobial resistance of pathogens involved in uncomplicated urinary tract infections", INTERNATIONAL JOURNAL OF ANTIMICROBIAL AGENTS, ELSEVIER, AMSTERDAM, NL, vol. 34, no. 5, 1 November 2009 (2009-11-01), pages 407-413, XP026601918, ISSN: 0924-8579, DOI: 10.1016/J.IJANTIMICAG.2009.04.012 [retrieved on 2009-06-07]

## Description

The present invention relates to the fields of medicine and microbiology and particularly, to novel strains of *Lactobacillus plantarum* to be used as a probiotic to benefit human and animal health. The present invention includes improved probiotic formulation which is used as food product, food supplement, medical food, medicament, pharmaceutical product, veterinary product, and personal hygiene product, and uses thereof.

### BACKGROUND ART

### Probiotics

The concept of probiotic microorganisms was born from the hypothesis of the Nobel Prize Elie Metchnikoff, who suggested that the consumption of bacteria able to ferment (commonly lactic acid-producing bacteria) has a positive effect on the microbiota of the colon, reducing the presence of bacterial toxins and other microbial activities which have a negative impact on human health.

"Probiotics are live microorganisms which, when administered in adequate amounts, confer a health benefit on the host". Nowadays, there are a lot of references about the usefulness of probiotics to treat several gastrointestinal disorders. In addition, some studies suggest that probiotics may modulate the immune system, prevent allergies and related conditions.

Probiotic bacteria must fulfil different requirements related to lack of toxicity, viability, colonization capacity and beneficial effects. The properties of each bacterial strain are unique and cannot be extrapolated to other strains of the same species (ARAYA, M., et al., Guidelines for the Evaluation of Probiotics in Food - Joint FAO/WHO Working Group, FAO/WHO, Editor 2002, Food and Agriculture Organization of the United Nations and World Health Organization: Ontario, Canada). Therefore, it is important to find those strains that have a better performance in all probiotic requirements.

Although the concept of probiotics was associated with intestinal microbial flora, studies conducted as early as 1987 showed that this concept could also be extended to the urinary tract. A few scientific groups have been developing this idea for some years, which culminated in the identification of some strains of *Lactobacillus* which are useful to treat complications of the urinary tract [REID, G. et al. Examination of strains of lactobacilli for properties which may influence bacterial interference in the urinary tract. J. Urol. 1987, Vol. 138, pages 330-335].

### Cystitis or urinary tract infection (UTI)

### General information

Cystitis is an acute infection of urinary bladder or kidney, which is more common in women than in men. There are different clinical entities related to cystitis, namely, acute cystitis, acute pyelonephritis (or infection of kidney), uncomplicated UTI (or acute cystitis or pyelonephritis occurring in healthy premenopausal, non-pregnant women with no history of abnormal urinary tract), the so called complicated UTI (or infection in patients with functional, metabolic, or anatomical conditions -such as obstruction, pregnancy, diabetes, neurogenic bladder, renal insufficiency, immunosuppression- that may increase risk of treatment failure or serious outcomes), and recurrent UTI (rUTI) [HOOTON, T.M. Clinical practice. Uncomplicated urinary tract infection. N. Engl. J. Med. 2012, Vol. 366, No. 11, pages 1028-1037; GRABE, M. et al. European Association of Urology (EAU). Guidelines on urological infections. EAU. March 2013]. Recurrence of UTI (or recurrent UTI) occurs because of either a relapse or a reinfection. A relapse is where there has been failure to cure the original infection with (or without) treatment. Re-infection is where the original infection was cleared by treatment (e.g. antibiotics) but occurs again within weeks. A relapse is caused by the same infecting microorganism that was present before initial treatment, usually due to a resistance of the microorganism to the drug treatment. A reinfection is a new episode of bacteriuria with a microorganism that is different from the original one (e.g. bacteriuria with *S. saprophyticus* species when the original infection was caused by *E coli*), although a reinfection may also occur with the same organism that caused the original infection. In this later case, the main feature differentiating a reinfection from a relapse is that individual episodes of reinfection are usually separated by a symptom-free interval of at least a month after antibiotics are stopped and the urine has shown no bacterial growth. [SALVATORE, S. et al. Urinary Tract Infections in Women. Eur. J. Obstet. Gynecol. Reprod. Biol. 2011, Vol. 156, pages 131-136].

### Epidemiology

Regarding the incidence or prevalence of UTI, they are among the most common bacterial infections in women. It is estimated that about 40-60 % of premenopausal women would develop at least a UTI in their life. In general, the average estimated incidence per year of a UTI in females is 2.1 % in infancy, 3 % in childhood, 15.2 % for adult women aged 17-39 years, 11.4 % for premenopausal women (aged 40-59 years), and 9.7 % for postmenopausal women (aged 60-79 years) [FOXMAN, B. and BROWN. P. Epidemiology of urinary tract infections: transmission and risk factors, incidence, and costs. Infect. Dis. Clin. North. Am. 2003, Vol. 17, No. 2, pages 227-241]. In young sexually active women and in postmenopausal women, the incidence of uncomplicated UTI is estimated in 0.5-0.7 and 0.07 per person-year, respectively [HOOTON, T. M. et al. A prospective study of risk factors for symptomatic urinary tract infection in young women. N. Engl. J. Med. 1996, Vol. 335, No. 7, pages 468-474; JACKSON, S. L. et al. Predictors of urinary tract infection after menopause: a prospective study. Am. J. Med. 2004, Vol. 117, No. 12, pages 903-11].

In general terms, the prevalence of cystitis in men is lower than in women being more common in females than males during youth and adulthood. However, the prevalence of UTI in elderly men and women is nearly equal. An annual incidence of 6-8 UTI per 10,000 men aged 21-50 years is estimated [ULLERYD, P. et al. Febrile urinary tract infection in men. Int. J. Antimicrob. Agents. 2003, Vol. 22, Suppl. 2, pages 89-93].

### Etiology

According to the Infectious Diseases Society of America and the European Society for Microbiology and Infectious Diseases, in general, the microbial spectrum of uncomplicated cystitis and pyelonephritis in women consists of *Escherichia coli* (75-95 %) [GUPTA, K. et al. International clinical practice guidelines for the treatment of acute uncomplicated cystitis and pyelonephritis in women: A 2010 update by the Infectious Diseases Society of America and the European Society for Microbiology and Infectious Diseases. Clinical Infectious Diseases. 1 March 2011, Vol. 52, No. 1, e103-e120].

Although data can vary considerably depending on the population under study, in particular, on the country, and the gender and age, microorganisms such as *Staphylococcus saprophyticus, Proteus mirabilis* and *Klebsiella pneumoniae* are proposed as the most frequent second causative agents of UTI. For example, in UTI affecting Swedish women in the range of 15-29 years, *S. saprophyticus* has been proposed to be present in 14.7 % of UTI episodes, being especially relevant in sexually active women [ERIKSSON, A. et al. The relative importance of Staphylococcus saprophyticus as a urinary tract pathogen: distribution of bacteria among urinary samples analyzed during 1 year at a major Swedish laboratory. APMIS. 2012, Vol. 121, pages 72-78]. It has been also reported that *P. mirabilis* and *K. pneumoniae*, can be present in 3 % and 5.9 % of the cases in the overall Swedish population. In other European countries, data also support the relevance of these microorganisms as the most common secondary causative UTI agents [GARCíA-GARCíA, M.I. et al. In vitro susceptibility of community-acquired urinary tract pathogens to commonly used antimicrobial agents in Spain: a comparative multicenter study (2002-2004). J. Chemotherapy. 2007, Vol. 19, pages 263-270].

### Treatment of UTI

The most frequently recommended agents for treating uncomplicated UTI in women include the following antibiotics [GUPTA, K. *et al., supra;* GRABE, M. *et al., supra*]:
1.- co-trimoxazole, also known as trimethoprim-sulfamethoxazole (TMP-SMX), 160/800 mg (1 double-strength tablet) orally twice daily for 3 days;
2.- nitrofurantoin monohydrate/macrocrystals, 100 mg orally twice daily for 5-7 days; and,
3.- fosfomycin-trometamol, 3 g orally in single dose.

However, in general, antibiotic use leads to an increased presence of drug-resistant microorganisms, which is not an exception in the case of UTI.

For instance, the resistance ratios of TMP-SMX are increasing, especially outside of the United States. In fact, the European Association of Urology is no longer recommending it as the first-line treatment agent, especially if local resistance rates are ≤ 20 %, if the infecting strain is known to be susceptible to it, or if it has been used for the treatment of UTI in the previous 3 months. It is estimated that in Spain only 66.1 % of clinical isolates of *E. coli* are sensitive to this antibiotic [ANDREU, A., et al. Etiología y sensibilidad a los antimicrobianos de los uropatógenos causantes de la infección urinaria baja adquirida en la comunidad. Estudio nacional multicéntrico. Enferm. Infecc. Microbiol. Clin. 2005, Vol. 23, No. 1, pages 4-9].

On the other hand, nitrofurantoin cannot be considered a useful short-term treatment (up to 3 days) and it is only recommended for treatments between 5 to 7 days, which can compromise the adhesion to the treatment. Although the resistance to nitrofurantoin has been reported to remain low (3-11 %), it is considered not effective against microorganisms such as *P. mirabilis* and *Klebsiella* species [KAHLMETER, G. An international survey of the antimicrobial susceptibility of pathogens from uncomplicated urinary tract infections: the ECO.SENS Project. Journal of Antimicrobial Chemotherapy. 2003, Vol. 51, pages 69-76]. Moreover, the use of nitrofurantoin has been discussed somewhat controversially in Europe due to its potentially dangerous side effects after prolonged exposure (polyneuropathy, interstitial pneumonia and hepatitis) [HUMMERS-PRADIER, E. et al. Urinary tract infections in adult general practice patients. British Journal of general practice. 2002, Vol. 52, pages 752-761].

Therefore, fosfomycin-trometamol is commonly selected as first treatment choice due to its broad spectrum of action, and effectiveness against *E. coli.* On top of that it has the advantage that it is administered in a single dose, which assures high treatment adhesion rates, and consequently decreasing the probability of antibiotic resistances. However, a limitation in the use of fosfomycin-trometamol is that, in some populations, albeit being very effective against *E. coli,* the effectivity against other causative pathogens, such as S. *saprophyticus* strains, is low. For instance, GARCÍA-GARCÍA M.I., *et al. supra*, reported that the susceptibility rate of S. *saprophyticus* to fosfomycin in the Spanish population was only 56.3 % in 2002 and decreased to 20.6 % in 2004, while in the same period the susceptibility of *E. coli* was higher than 97 % [GARCÍA-GARCÍA, M.I., *et al. supra*]. Additionally, compared to *E. coli,* the activity of fosfomycin can be lower against other agents responsible of UTI, such as *P. mirabilis* (76 %) and *K. pneumoniae* (78.5 %). This reduced efficacy can be attributed to an increase in antibiotic resistance of these uropathogens, which can be expected to increase in the next years.

In summary, both the lifetime risk of UTI and the incidence of recurrent UTI episodes, mainly among women, are high. In general, antibiotic use leads to the increased presence of drug-resistant microorganisms, which is not an exception in the case of UTI. In fact, as discussed above, there is the problem of increasing UTI main causative pathogens showing resistance to the first-line empiric agents for acute uncomplicated cystitis, namely fosfomycin and TMP-SMX. Also as already discussed, rUTI episodes necessitate in most of the cases repeated antimicrobial treatment courses. In fact, long-term antibiotic prophylaxis is the most common method for currently managing rUTI, with the important limitation of the mentioned increasing antimicrobial resistances.

All these factors make treatment and prevention of UTI more and more problematic. Consequently, novel, safe, and effective non-antibiotic prevention and treatment strategies alone, or in combination with antibiotics are currently needed.

### Alternative treatments for the management of UTI

The art describes other products that are currently used for the prevention and treatment of rUTI, such as the cranberry and lingonberry extracts. Such berries are rich in proanthocyanidins (PACs), i.e. tannins able to prevent the expression of the P fimbriae of the uropathogen *E. coli,* inhibiting bacterial cell wall synthesis and cellular expression of adhesion molecules to the urogenital mucosa. Some findings indicate that cranberry-containing products could be clinically associated with protective effect against UTI [SENGUPTA, K. et al. A Randomized, double blind, controlled, dose dependent clinical trial to evaluate the efficacy of a proanthocyanidin standardized whole cranberry (Vaccinium macrocarpon) powder on infections of the urinary tract. Current Bioactive Compounds. March 2011, Vol. 7, No 1, pages 39-42]. However, these observations are not supported by results in some clinical trials [BARBOSA-CESNIK, C. et al. Cranberry juice fails to prevent recurrent urinary tract infection: results from a randomized placebo-controlled trial. Clinical Infectious Diseases. 2011, Vol. 52, No. 1, pages 23-30]. So overall scientific evidence is far from being conclusive.

Lactobacilli are present in the vaginal flora developing various functions essential for the maintenance of the vaginal physiological equilibrium and preventing infections. The "normal" flora of the reproductive tract is numerically dominated by *Lactobacillus* species that maintain the natural acidic pH of the vagina, act as a natural barrier against pathogens and produce antimicrobial compounds that inhibit the colonization and/or overgrowth of pathogenic microorganism. However, it is recognized that the composition of the microbiota can vary from day to day even in women without indication of infections, due to the influence of several factors such as: hormonal changes (i.e. menstrual cycles), biomaterial-oriented contraception, sexual contact, feminine hygiene practices, antibiotic consumption, nutritional status, microbial interspecies competition or commensalism. Urogenital conditions in women are often characterized by an alteration in the local flora from predominance of lactobacilli to opportunistic pathogens. Urogenital conditions include vaginosis, vaginitis or UTI. For instance, bacterial vaginosis is a common condition characterized by a shift in the vaginal microbiota from protective lactobacilli to pathogenic bacteria such as *Gardnerella vaginalis* and *Mycoplasma hominis.* Similarly, vaginitis is an alteration of the vaginal flora, usually caused by an overgrowth or infection of *Candida* or *Trichomonas* species. Vaginitis in comparison with vaginosis further results in a vulvovaginal inflammation.

It has been postulated that lactobacilli may be used for the treatment of vaginal conditions. For example, probiotic formulations have been studied for its use in the treatment of bacterial vaginosis (BV) [LARSSON, P.G. et al. Extended antimicrobial treatment of bacterial vaginosis combined with human lactobacilli to find the best treatment and minimize the risk of relapses. BMC Infectious Diseases. 2011. Vol. 11, page 223]. The study shows that aggressive treatment of the patient with repeated antibiotic treatment with clindamycin and metronidazole together with vaginal gelatine capsules containing different strains of lactobacilli, can provide long lasting cure. However, in this study there is no control without probiotic, so differences in efficacy and in risk of relapse between the combined treatment and the use of antibiotics as usual, cannot be evaluated.

However, uncertainty exists as to whether the vaginal application of lactobacilli reduces the infection rate in cystitis-prone women [BAERHEIM, A. et al. Vaginal application of lactobacilli in the prophylaxis of recurrent lower urinary tract infection in women. Scand. J. Prim. Health Care. 1994. Vol. 12, pages 239-243].

AQUILEA INTIMUS is an oral probiotic composed by two strains of lactobacilli, i.e. *Lactobacillus rhamnosus* GR-1®, isolated from the urethra of a healthy woman, and *Lactobacillus reuteri* RC-14®, isolated from the vagina of a healthy woman. These two probiotic strains have been demonstrated again to be effective for treating bacterial vaginosis [MARTINEZ, R.C.R. et al. Improved cure of bacterial vaginosis with single dose of tinidazole (2 g), Lactobacillus rhamnosus GR-1, and Lactobacillus reuteri RC-14: a randomized, double blind, placebo control trial. Canadian Journal of Microbiology. 2009, Vol. 55, pages 133-138]. Another study reported that an oral composition comprising the same strains augmented the effect of metronidazole therapy for bacterial vaginosis [ANUKAM, K. et al. Augmentation of antimicrobial metronidazole therapy of bacterial vaginosis with oral probiotic Lactobacillus rhamnosus GR-1 and Lactobacillus reuteri RC-14: randomized, double-blind, placebo controlled trial. Microbes and infection. 2006. Vol. 8. pages 1450-1454].

Nevertheless, these strains failed to demonstrate efficacy in the reduction of the incidence of rUTI as reported in a randomized, double-blind, noninferiority trial in 252 postmenopausal women with rUTI [BEEREPOT, M.A. et al. Lactobacilli vs antibiotics to prevent urinary tract infections: a randomized, double-blind, noninferiority trial in post-menopausal women. Archives of Internal Medicine. 2012, Vol. 172, No. 9, pages 704-712]. As shown in Table 2 of the referenced article, no statistically significant difference was observed between the incidence of rUTI in women after 12 months of prophylaxis with *L. reuteri* RC-14® and *L. rhamnosus* GR-1® vs prophylaxis with TMP-SMX. The strain *Lactobacillus rhamnosus* GR-1® is also disclosed in the European patent EP 1137423 B1, in a probiotic composition together with at least another *Lactobacillus* probiotic strain, namely *Lactobacillus fermentum* B-54 (ATCC55884). In particular, EP 1137423 B1 illustrates how oral ingestion of lactobacilli by a women presenting chronic symptomatic UTI can reduce the presence of *Enterococcus faecalis* in urine and vaginal swaps. However, the study was conducted in only one person and there was no control group consuming placebo. Moreover, there is no information of whether the administration of lactobacilli can reduce the presence of main causative agents of UTI, namely *E. coli, S. saprophyticus* and/or *P. mirabillis.* Therefore, the efficacy of lactobacilli composition for treating UTI remains questionable.

The art also discloses the results of a case study, without placebo group, carried on 9 women and of a randomized, placebo-controlled phase 2 trial with 100 women with history of rUTI (i.e. one or more episodes of UTI in the preceding 12 months) for evaluating the safety and effectiveness of *Lactobacillus crispatus* strain GAI 98332 vaginal suppositories [UEHARA, S. et al. A pilot study evaluating the safety and effectiveness of Lactobacillus vaginal suppositories in patients with recurrent urinary tract infection. International Journal of Antimicrobial Agents. 2006. Vol. 28, No. 1, pages S30-S34; STAPLETON, A.E. et al. Randomized, placebo-controlled phase 2 trial of a Lactobacillus crispatus probiotic given intravaginally for prevention of recurrent urinary tract infection. Clin. Infect. Dis. May 2011. Vol. 52, No. 10, pages: 1212-1217, respectively]. Strain GAI 98332 of *L. crispatus* was isolated from the vagina of healthy women. In both studies, a significant reduction was observed in the number of rUTI caused by *E. coli.* However, both studies are silent regarding recurrences caused by other UTI pathogens as well as the antibiotic used for the conventional treatment.

Another marketed probiotic is Isadin α Barcilus®, a soft capsule vaginal probiotic with emollient and lubricating excipients, used in patients with vulvovaginal candidiasis and vaginal infections of varying causes. Isadin α Barcilus® is composed by *Lactobacillus plantarum* P17630 strain. No reports have been found regarding its efficacy for treating UTI.

Therefore, although it could be expected that lactobacilli strains can be effective in the prevention and treatment of vaginal conditions, their effectiveness in the management of UTI is far from have been demonstrated.

### DETAILED DESCRIPTION OF THE INVENTION

The problem to be solved by the present invention is to provide compositions and remedies useful for the prevention and/or treatment of urinary tract infections.

As mentioned above, the prior art has described probiotic strains that can be useful for treating urogenital conditions such as bacterial vaginosis, but not UTI. Surprisingly, the inventors have found lactic acid bacteria strains that are useful for the management of urinary tract infections and can also be combined with conventional antimicrobial treatments.

The invention is as defined in the claims. Thus, in one aspect, the present invention provides a composition for use in the prevention and/or treatment of urinary tract infections comprising (i) an effective amount of at least one antimicrobial agent which is active against pathogens of the urogenital tract, and (ii) an effective amount of at least one lactic acid bacteria strain, wherein the strain has non-transmissible resistance to the antimicrobial agent and wherein (i) and (ii) are formulated in a single or in separate administration forms.

The term "effective amount" (or "therapeutically effective amount") as used herein, means an amount of agent high enough to deliver the desired benefit, i.e. prevent, delay the onset of, or reduce the progression of, but low enough to avoid serious side effects within the scope of medical judgment, administered alone or in combination with an additional therapeutic agent to a cell, tissue, or subject. When applied to an individual active ingredient (or active agent) administered alone, a therapeutically effective dose refers to that ingredient alone. When applied to a combination, a therapeutically effective dose refers to combined amounts of the active ingredients that result in the therapeutic effect, whether administered in combination, serially or simultaneously.

The term "antimicrobial" has in the present invention the normal meaning in the field, namely, an agent that kills microorganisms or inhibits their growth. They can be further grouped according to the microorganisms they act primarily against, e.g. against bacteria (antibacterials) and against fungi (antifungals).

"Resistance to antimicrobial agent" is understood as the capacity of a microorganism to grow in the presence of the antimicrobial agent. In general, resistance may take the form of a spontaneous or induced genetic mutation, or the acquisition of resistance genes from other bacterial species by horizontal gene transfer via conjugation, transduction, or transformation. The resistance is considered "non-transmissible", as used herein, when the potential risk of lateral or horizontal spread of this resistance to other microorganism is minimal.

In a particular embodiment, the at least one antimicrobial agent comprises a compound suitable for preventing and/or treating UTI. Agents for preventing and/or treating UTI are antibiotics and natural ingredients with antimicrobial effect. Antibiotics of common choice for treating UTI are, without limitation, nitrofurantoin, trimethoprim-sulfamethoxazole, pivmecillinam, fosfomycin, nitrofurantoin, quinolones, tetracyclines, penicillins and beta-lactams. In a preferred embodiment, the at least one antimicrobial agent is fosfomycin. In a preferred embodiment, the at least one antimicrobial agent is a fluoroquinonolone and, particularly, norfloxacin.

In another particular embodiment, the at least one antimicrobial agent is a natural product or ingredient for treating UTI. Natural products and ingredients for treating UTI include, without limitation, extracts, juices and isolated compounds from plant materials, e.g. fruits and herbs. In a particular embodiment natural ingredient is obtained from cranberries (*Vaccinum macrocarpon*), bilberry (*V. myrtilis*), European cranberry (*V. oxycoccus*), blueberries (*V. corymbosum*), lingonberry (*V. vitis-idaea*), other herbal or fruit and grape extracts and juices (e.g. from coffe, tea, hibiscus, *Orthoshipon* sp., *Juniperus* sp., *Aloe* sp., salvia, *Equisetum* sp., *Lavandula* sp., *betula,* mannose), berberine, arbutin and/or vitamins, particularly, vitamin C or B.

In a particular embodiment, the at least one lactic acid bacteria strain has the ability to antagonize the urogenital pathogen *Staphylococcus saprophyticus* wherein the ability of the strain to antagonize the urogenital pathogen is determined by the following steps:
(i) swabbing uniformly urogenital pathogen strain in plates containing Trypticase Soy Agar medium and growing to confluence in a CO₂ incubator at 37 °C and 5 % of CO₂;
(ii) placing two 6 mm diameter cylinder sections of a uniformly seeded confluent agar plate of the cells in contact with the urogenital pathogen seeded plate, confronting both (a) the grown side of one-cylinder section against the urogenital pathogen seeded plate; and (b) the non-grown side of the other cylinder section against the urogenital pathogen seeded plate; and incubating overnight at 37 °C;
(iii) measuring next day the inhibition zones by placing the agar plate over a flat rule; and,
(iv) calculating the growth inhibitory activity (GI) by subtracting the cylinder diameter (CD) from the inhibition zone diameter (IZD) measured in centimeters and dividing this difference by 2, following the formula GI = (IZD-CD) / 2.

The term "inhibition zone" as used herein, refers to a zone around the lactic acid bacteria strain of interest where the growth of pathogen is partially or totally reduced compared to the growth of the pathogen in the absence of the lactic acid bacteria strain of interest.

It is considered that lactic acid bacteria strains have the ability to antagonize the urogenital pathogen *Staphylococcus saprophyticus*, wherein this ability is determined by the steps described above, when the lactic acid bacteria strain has a growth inhibitory (GI) activity which is measurable by visual inspection. In a particular embodiment, the GI of the lactic acid bacteria of interest is 0.5 mm. In other particular embodiments, the GI is 1 mm, 2 mm, 3 mm or higher.

Pathogens other than *S. saprophyticus* are known to be main causative agents of urogenital tract infections, i.e. *Proteus mirabilis, Escherichia coli* and *Klebsiella pneumonia.* The composition of the invention is also useful for preventing and/or treating urinary tract infections caused by these pathogens.

Thus, in a particular embodiment the at least one lactic acid bacteria strain has also the ability to antagonize the urogenital pathogens *Proteus mirabilis, Escherichia coli or Klebsiella pneumonia.* Working EXAMPLE 4 herein provides detailed description of an assay suitable to measure the capacity of the lactic acid bacteria strain of interest to antagonize these pathogens.

In an even more preferred particular embodiment the at least one lactic acid bacteria strain has also the ability to antagonize the urogenital pathogens *Proteus mirabilis, Escherichia coli and Klebsiella pneumonia.* Working EXAMPLE 4 herein provides detailed description of an assay suitable to measure the capacity of the lactic acid bacteria strain of interest to antagonize these pathogens.

It is preferred that the lactic acid bacteria strain as discussed herein is an isolated lactic acid bacteria strain.

As understood by the skilled person in the present context - the term "isolated" relates to that the lactic acid bacteria strain is isolated from its natural environment - i.e. it is not present in its natural environment.

Based on the detailed assay described herein (see EXAMPLE 4 for the antagonistic activity assay) the skilled person is routinely able to repeat this assay to objectively determine whether a lactic acid bacteria strain of interest antagonize the urogenital pathogens. It is relevant to note that the descriptions and conditions of the antagonistic activity assay disclosed in steps (i)-(iv) above and in EXAMPLE 4 are not limiting the scope of the invention. The assay is one suitable to test the ability of the lactic acid bacteria strains of interest to antagonize the urogenital pathogens *Staphylococcus saprophyticus, Proteus mirabillis, Escherichia coli* or *Pseudomonas pneumoniae.* It is known by the skilled in the art that pathogens may show different tolerance to the inhibitory activity of the lactic acid bacteria depending on the pathogen strain used for performing assays and its resistance. Thus, the scope of the invention includes lactic acid bacteria strains showing an inhibitory activity against any strain of *Staphylococcus saprophyticus, Proteus mirabillis, Escherichia coli* or *Pseudomonas pneumoniae.*

The lactic acid bacteria strain is *Lactobacillus plantarum* strain selected from the group consisting of: strain deposited in the Spanish Type Culture Collection (CECT) under the accession number CECT8675, strain deposited under the accession number CECT8676, strain deposited under the accession number CECT8677; strain deposited under the accession number CECT8678, and a mutant of any of the deposited strains, wherein the mutant has been obtained using the deposited strain as starting material and applying mutagenesis, and wherein the obtained mutant retains or enhances at least the ability of the deposited strain to antagonize the urogenital pathogen *Staphylococcus saprophyticus.* In particular embodiments, the composition comprises combinations of two, three, or four strains, selected from the group consisting of: CECT8675 strain, CECT8676 strain, CECT8677 strain, CECT8678 strain and mutants thereof. In still another particular embodiment, the composition comprises an effective amount of *L. plantarum* CECT8675 and *L. plantarum* CECT8677.

The inventors provide new strains of lactic acid bacteria, particularly from *Lactobacillus* genus. Provision of these strains are the result of extensive studies of different lactic acid bacteria strains isolated from healthy humans. *Lactobacillus plantarum* strain F2032, *Lactobacillus plantarum* strain F3164, *Lactobacillus plantarum* strain F1034 and *Lactobacillus plantarum* strain F2070 were deposited on 17-July-2014, 15-July-2014, 15-July-2014 and 15-July-2014, respectively, in the Spanish Type Culture Collection (Colección Española de Cultivos Tipo, CECT, Edificio 3 CUE, Parc Científic, Universitat de Valencia, Catedrático Agustín Escardino, 9, 46980-Paterna, Valencia, Spain), by the depositor AB-Biotics, S.A., sited at Parc de Recerca UAB, Edifici Eureka, Campus UAB, office P2M1, 08193-Bellaterra (Spain). The strains of *L. plantarum* received the accession numbers CECT8677, CECT8675, CECT8678 and CECT8676, respectively after the International Authority of Deposit declared the strain as viable.

The strains of the present invention were selected because of the following distinguishing properties/capacities:
- The capacity to inhibit the growth of *S. saprophyticus,* thus displaying a broader spectrum activity compared to other commercially available probiotics to help managing UTI, especially wherein the causative agent is this pathogen. As indicated in the background art and the EXAMPLES section, some lactobacilli strains have been used for the treatment and/or prevention of UTI. However, said strains display no significant antagonistic activity against several isolates of *S. saprohyticus* (see EXAMPLE 4.1). Surprisingly, strains CECT8677, CECT8675, CECT8678 and CECT8676 showed remarkably antagonistic activity against the three strains of S. *saprophyticus* tested. Thus, as supported by the data disclosed in EXAMPLE 4.1, the capacity of inhibiting *S. saprohyticus* is not an inherent feature of the genus *Lactobacillus,* not even of lactobacilli strains of urethral or vaginal origin, such as commercial strains *L. rhamnosus* GR-1® *and L. reuteri* RC-14®, respectively, and much less an inherent feature *of L. plantarum* strains such as *L. plantarum* P17630, a probiotic commercial strain. Thus, the present invention provides lactic acid bacteria strains having the ability to antagonize *Staphylococcus saprophyticus.*

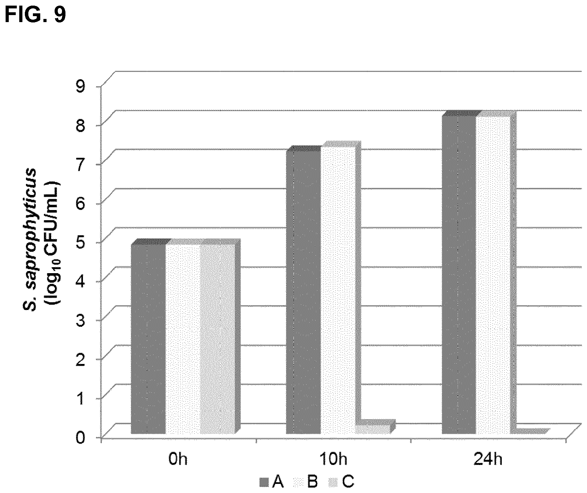

- The capacity to inhibit the growth of other urinary tract pathogens, namely, *Proteus mirabilis, Escherichia coli and Klebsiella pneumoniae*, thus displaying an even broader spectrum activity against uropathogens compared to other commercially available probiotics to help managing UTI. As already mentioned above, some *Lactobacillus* strains have been used for the treatment and/or prevention of UTI. However, said strains display no significant antagonistic activity against isolates of *P. mirabilis, E. coli* and *K. pneumoniae* (see EXAMPLES 4.2-4.3). Surprisingly, strains CECT8677, CECT8675, CECT8678 and CECT8676 showed remarkably antagonistic activity against them. Thus, as supported by the data disclosed in EXAMPLES 4.2-4.3, the capacity of inhibiting *P. mirabilis, E. coli and K. pneumoniae* is not an inherent feature of the genus *Lactobacillus,* not even of lactobacilli strains of urethral or vaginal origin, such as commercial strains *L. rhamnosus* GR-1® and *L. reuteri* RC-14®, respectively, and much less an inherent feature of *L. plantarum* strains such as *the* probiotic commercial *L. plantarum* P17630.
- The capacity to survive at high concentrations of fosfomycin, antibiotic typically used for managing UTI. As it is shown in EXAMPLE 5, the growth of strains CECT8677, CECT8675, CECT8678 and CECT8676 is not inhibited even using saturated concentrations of fosfomycin. In other words, even when using high concentrations of fosfomycin together with said strains, the efficacy of the resulting combination is not compromised and, therefore, it can exert both the probiotic and the antimicrobial functions. In summary, the strains of the present invention can be co-administered with the antibiotic fosfomycin.
- The capacity to survive at high concentrations of other antibiotics used for treating UTI such as norfloxacin (EXAMPLE 5).
- The capacity to survive in the presence of natural active ingredients commonly prescribed for treating UTI such as cranberry and/or lingonberry extracts, thus allowing the bacterial strains of the present invention to be co-administered with said active UTI ingredients (see EXAMPLE 6).
- An improved capacity to aggregate, as shown in EXAMPLE 7, which is one of the initial steps leading to the formation of protective biofilms against (uro)pathogens. Biofilm allows forming a protective barrier or 'film' which makes difficult the colonization of (uro)pathogens due to competence for adhesion sites.
- The capacity to produce short chain fatty acids (SCFA), as shown in EXAMPLE 8. Production of SCFA from non-digestible fibers is an interesting probiotic trait. This trait is desirable in a probiotic because the produced SCFA show several beneficial properties in the host. Among their various properties, there is very good evidence supporting that increasing the concentrations of SCFA enhances the growth of protective bacteria while limiting the growth of pathogens, and it can improve intestinal barrier function, especially in the case of butyrate [KOBOZIEV, I. et al. Role of the enteric microbiota in intestinal homeostasis and inflammation. Free Radical Biology & Medicine. 2014, Vol. 68, pages 122-133]. As a result, SCFA can ameliorate the symptoms associated to adverse gastrointestinal conditions such as diarrhea which is an important risk factor of cross-contamination of intestinal pathogens to the vagina that favors vaginal infections and UTI.
- Tolerance to the environmental conditions of the gastrointestinal and genitourinary tracts, and thus ability to exert their beneficial effect in the desired locations of the body, as shown in EXAMPLE 9.
- The capacity of the probiotic strains of the present invention to increase the efficacy of fosfomycin for inhibiting pathogens associated with UTI. As shown in EXAMPLE 10 and FIG. 9, the combination of fosfomycin with the strains of the present inventions, significantly reduces the concentration of *Staphylococcus saprophyticus.* Therefore, the combined use of antibiotic and probiotic can be considered an effective management option for treating *S. saprophyticus* infections.
- A wide variety of lactic acid bacterial species have a long history of apparent safe use. The European Food Safety Authority has developed a system granting the "Qualified Presumption of Safety" (QPS) status to taxonomical units with a proven long history of apparent safe use. The strains of the invention belong to a bacterial species that has QPS status (ANDREOLETTI, O. et al. The maintenance of the list of QPS microorganisms intentionally added to food or feed. Question no: EFSA-Q-2008-006. The EFSA Journal. 2008, Vol. 923, pages 1-48).

In summary, it is believed that no prior art describes *Lactobacillus* strains and particularly *Lactobacillus plantarum* strains with the above-mentioned features. It is noteworthy that the strains of *Lactobacillus plantarum* CECT8677, CECT8675, CECT8678 and CECT8676 meet all the previously mentioned properties together, being therefore suitable for treating UTI.

Remarkably, all the strains of the invention have the ability to antagonize all the following urogenital pathogens *Staphylococcus saprophyticus, Escherichia coli, Klebsiella pneumoniae* and *Proteus mirabilis.* and can be combined with antimicrobial agents, i.e. antibiotics, routinely used for treating UTI. Moreover, the strain of the invention had good tolerance and can be combined with alternative treatments based on natural products and ingredients, e.g. juices and isolated compounds from plant materials (fruits and herbs) such as those belonging to *Vaccinum* genus (e.g. cranberry); and vitamins (e.g. Vitamin C)

In another aspect, the present invention provides a composition comprising an effective amount of at least one strain of *Lactobacillus plantarum,* wherein the strain has the ability to antagonize the urogenital pathogen *Staphylococcus saprophyticus,* and wherein the ability of the strain of *L. plantarum* to antagonize the urogenital pathogens is determined by the following steps: (i) swabbing uniformly urogenital pathogen strains in plates containing Trypticase Soy Agar medium and growing to confluence in a CO₂ incubator at 37 °C and 5 % of CO₂; (ii) placing two 6 mm diameter cylinder sections of a uniformly seeded confluent agar plate of the *L. plantarum* cells in contact with the urogenital pathogen seeded plate, confronting both (a) the grown side of one cylinder section against the urogenital pathogen seeded plate; and (b) the non-grown side of the other cylinder section against the urogenital pathogen seeded plate; and incubating overnight at 37 °C; (iii) measuring next day the inhibition zones by placing the agar plate over a flat rule; and, (iv) calculating the growth inhibitory activity by subtracting the cylinder diameter (CD) from the inhibition zone diameter (IZD) measured in centimeters and dividing this difference by 2, following the formula GI = (IZD-CD) / 2.

In a particular embodiment, the growth inhibitory activity (GI) of the at least one strain of *Lactobacillus plantarum* is 0.5 mm. In other particular embodiments, the GI is 1 mm, 2 mm, 3 mm or higher.

In a particular embodiment, the at least one strain of *Lactobacillus plantarum,* has also the ability to antagonize the urogenital pathogens *Proteus mirabilis, Escherichia coli or Klebsiella pneumoniae*, wherein the ability of the strain to antagonize the urogenital pathogens is determined by the steps as defined above.

In an even more preferred particular embodiment, the at least one strain of *Lactobacillus plantarum,* has also the ability to antagonize the urogenital pathogens *Proteus mirabilis, Escherichia coli* and *Klebsiella pneumoniae,* wherein the ability of the strain to antagonize the urogenital pathogens is determined by the steps as defined above.

As discussed above, it is preferred that the lactic acid bacteria strain discussed herein is an isolated lactic acid bacteria strain - i.e. it is preferred that the *Lactobacillus plantarum* as discussed herein is an isolated lactic acid bacteria strain.

In a particular embodiment, the present invention provides the composition of the previous aspect, wherein the at least strain of *L. plantarum* is selected from the group consisting of: CECT8675 strain, CECT8676 strain, CECT8677 strain, CECT8678 strain and mutants thereof, wherein the mutant has been obtained using the deposited strain as starting material and applying mutagenesis, and wherein the obtained mutant retains or enhances at least the ability of the deposited strain to antagonize the urogenital pathogen *Staphylococcus saprophyticus.* In a more particular embodiment, the at least one strain of *Lactobacillus plantarum* is selected from the group consisting of: CECT8675 strain, CECT8676 strain, CECT8677 strain, and CECT8678 strain. In particular embodiments, the composition comprises combinations of two, three, or four strains, selected from the group consisting of: CECT8675 strain, CECT8676 strain, CECT8677 strain, CECT8678 strain and mutants thereof. In still another particular embodiment, the composition comprises an effective amount of *L. plantarum* CECT8675 and *L. plantarum* CECT8677.

The general use of strains is in the form of viable cells. However, it can also be extended to non-viable cells such as killed cultures or cell lysates (obtained by e.g. exposure to altered pH, sonication, radiation, temperature, pressure, or among other means of killing or lysing bacteria) or compositions containing beneficial factors produced by any of the strains.

The strains of the invention are produced by cultivating (or fermenting) the bacteria in a suitable artificial medium and under suitable conditions. By the expression "artificial medium" for microorganisms is to be understood a medium containing natural substances, and optionally synthetic chemicals such as the polymer polyvinyl alcohol which can reproduce some of the functions of serums. The artificial medium is appropriately sterilized once the substances are mixed. Accordingly, among the natural substances, common suitable artificial media are nutrient broths that contain the elements including a carbon source (e.g. glucose), a nitrogen source (e.g. amino acids and proteins), water and salts needed for bacterial growth. Growth media can be liquid form or often mixed with agar or other gelling agent to obtain a solid medium. Growth media can be also considered any food matrix where bacteria can growth under appropriate conditions, such as the ingredients to make yoghurt.

The strains can be cultivated alone to form a pure culture, or as a mixed culture together with other microorganisms, or by cultivating bacteria of different types separately and then combining them in the desired proportions. After cultivation, and depending on the final formulation, the strains may be used as purified bacteria, or alternatively, the bacterial culture or the cell suspension may be used, either as such or after an appropriate post-treatment. In this description, the term "biomass" is understood the bacterial strains culture obtained after cultivation (or fermentation as a term synonymous to cultivation).

By the term "post-treatment" is to be understood in the context of the present invention, any processing carried out on the biomass with the aim of obtaining storable bacterial cells. The objective of the post-treatment is decreasing the metabolic activity of the cells in the biomass, and thus, slowing the rate of cellular deleterious reactions. As a result of the post-treatment, the bacterial cells can be in solid or liquid form. In solid form, the stored bacterial cells can be a powder or granules. In any case, both the solid and liquid forms containing the bacterial cells are not present in the nature, hence, are not naturally-occurring, since they are the result of artificial post-treatment process(es). The post-treatment processes may in particular embodiments require the use of one or more of so called post-treatment agent. In the context of the present invention, the expression "post-treatment agent" refers to a compound used to perform the herein described post-treatment processes. Among the post-treatment agents are to be included, without limitation, dehydrating agents, bacteriostatic agents, cryoprotective agents (cryoprotectants), inert fillers (also known as lyoprotectants), carrier material (also known as core material), etc., either used alone or in combination.

There are two basic approaches to decrease the metabolic activity of the bacterial cells, and thus, two approaches to carry out the post-treatment. The first one is decreasing the rate of all chemical reactions. This can be done lowering the temperature by means of refrigerating or freezing using refrigerators, mechanical freezers, and liquid nitrogen freezers. Alternatively, decreasing the rate of all chemical reactions can also be achieved by adding substances that inhibit the growth of the bacterial cells, namely a bacteriostatic agent, abbreviated Bstatic, which is a biological or chemical agent that stops bacteria from reproducing, while not harming them otherwise, among them the bacteriostatic antibiotics and preservatives. This is in contrast to bactericides, which kill bacteria. The skilled person knows the appropriate bacteriostatic to use. As example of bacteriostatic, and without limitation, bacteriostatic antibiotics which limit the growth of bacteria by interfering with bacterial protein production, DNA replication, or other aspects of bacterial cellular metabolism, in the adequate concentrations to keep only their bacteriostatic activity. A bacteriostatic agent can also be understood as any agent that is added to modify the environmental conditions such as pH, salts concentration, or the redox state.

The second approach to carry out the post-treatment is to remove water from the biomass, a process which can involve sublimation of water using a lyophilizer. Suitable techniques to remove water from the biomass are drying, freeze-drying, spray-drying or fluid bed-drying.

Post-treatments that result in solid form may be drying, freezing, freeze-drying, fluid bed-drying, or spray-drying.

The post-treatment after cultivation of the strains may consist in freezing. In this case, one or more cryoprotective agents must be added. The expressions "cryoprotective agent (or additive)" and "cryoprotectant" are used indistinctively in the present invention, and they refer to chemicals that protect the cells during freezing while minimize at the same time the detrimental effects of increased solute concentration and ice crystal formation that cause a drop in the number of viable cells and consequent losses in industrial production. The most commonly used cryoprotective agents are dimethylsulfoxide (DMSO) and glycerol, either alone or in combination; although other cryoprotectants that have been used occasionally, either alone or in combination, include: polyethylene glycol, propylene glycol, glycerine, betaine (or glycine betaine), polyvinylpyrolidone, sorbitol, dextran and trehalose. Cryoprotectants classified by families are: sulphoxides, monohydric alcohols and derivatives, diols and derivatives, triols, polyalcohols, mono-,di-,tri-,poly-saccharides, amides, N-alkylamides, imides, heterocyclic compounds, amino acids and carbonic acids, proteins, peptides, polypeptides, glycoproteins, and non-ionic surfactants. Protocols for freezing bacteria and the selection of suitable cryoprotectants are of choice and knowledge of the skilled in the art. Basically, bacteria can be conveniently cultivated or harvested from agar slants or plates, or from broth culture and harvested by centrifugation. In either case, cells are generally suspended in fresh growth medium containing the cryoprotective agent(s). Bacteria can also be concentrated by centrifugation, but are often suspended in the used medium and then diluted by adding an equal volume of fresh growth medium containing the cryoprotective agent(s). Then, the mix of cells and cryoprotectant(s) is left during at least 15 minutes but not longer than 45-60 minutes to equilibrate before the cooling process. The suspension is dispensed into vials and cooled at appropriate cooling rate.

When the post-treatment after cultivation of the strains is drying, it basically consists in drying in a desiccator *in vacuo* over dehydrating agents such as H₂SO₄ or P₂O₅, the microorganisms suspended in culture fluid or resuspended in saline.

The post-treatment after cultivation of the strains may consist in freeze-drying, as a method for removing water. Freeze-drying involves the removal of water from frozen bacterial suspensions by sublimation under reduced pressure. Lyophilized cultures are best maintained at 4 °C or lower. Sublimation occurs when a frozen liquid goes directly to a gaseous state without entering a liquid phase. The freeze-drying process results in a stable, readily rehydrated product. This process consists of three steps: pre-freezing the product to form a frozen structure, primary drying to remove most water, and secondary drying to remove bound water. Means and methods for freeze-drying are known in the art and at the skilled person's disposal. Due to objective and expected variability of industrial processes for manufacturing and isolation of lyophilized bacterial cultures, the latter commonly contain certain amount of inert filler also known as lyoprotectant. Its role is to standardize the content of live probiotic bacteria in the product. The following inert fillers in commercially available lyophilized cultures are used: sucrose, saccharose, lactose, trehalose, glucose, maltose, maltodextrin, corn starch, inulin, and other pharmaceutically acceptable non-hygroscopic fillers. Optionally, other stabilizing or freeze-protecting agents like ascorbic acid, are also used to form a viscous paste, which is submitted to freeze-drying. In any case, the so-obtained material can be grinded to appropriate size, including to a powder.

The post-treatment after cultivation of the strains may consist in spray-drying. Spray-drying is a process whereby a liquid formulation is converted into a dry powder in a single step. The process is typically performed by first atomizing the solution into artificially fine droplets as small as possible, maximising heat transfer and the rate of water vaporisation. Droplet sizes can range from 20 to 180 µm depending on the nozzle. There are two main types of nozzles: high pressure single fluid nozzle (50 to 300 bars) and two-fluid nozzles: one fluid is the liquid to dry and the second is compressed gas (generally air at 1 to 7 bars). The droplets are then dried quickly in a large chamber by using warm gas. The resulting dry particles are collected with a cyclone.

The post-treatment after cultivation of the strains may consist in fluid bed-drying. In the fluidized bed process first of all a carrier material (also known as core material) is needed as a basis for the substrate that should be dried. The carrier material may be, without limitation, cellulose. This carrier material is held up in the air to enable the application of the substrate via a spray nozzle. Compared to spray-drying the temperatures are lower. The bacteria are sprayed onto the dispersed carrier material in form of fine droplets. The contained water deprived by the dry air flows bottom-up through the fluidized bed. In the same time the stream of air is responsible for the fluidization of particles. Afterwards a protective coating is applied in the same matter onto the carrier that is coated with bacteria. The protective coating used must prevent bacteria cellular injury by stabilising the cell membrane constituents, e.g. by creating a porous structure in the dried product that makes rehydration easier and contains proteins that provide a protective coating for the cells. The protective coating material may be, without limitation, skim milk and calcium alginate. The structure of an encapsulated product achieved by fluidized bed coating consists of at least three parts: inside the (spherical) carrier or core material, uniform protective coating layer, on the outside, and in between the coated active ingredient of interest, namely, the bacteria. Sometimes multiple layers of different coating materials are required to obtain the desired product properties. The size of the resulting microcapsules varies between 200 µm and 2 mm.

Alternatively to having biomass preserved in solid form, biomass may be also preserved in liquid form. This may be done by adding a bacteriostatic agent as described above to stop bacteria growth to the culture medium or with an intermediate step of harvesting cells, resuspending the pellet in saline solution with a bacteriostatic agent, and optionally refrigerating it.

Sometimes, as described for instance above in the fluid bed-drying process, the probiotic preparation is subjected to an immobilisation and/or coating, or encapsulation process in order to improve the shelf life and/or functionalities. Several techniques for immobilisation, coating or encapsulation of bacteria are known in the art.

Accordingly, a particular embodiment of the present invention relates to at least one strain as defined above, hence, including the *Lactobacillus plantarum* CECT8677 or a mutant thereof, the *L. plantarum* CECT8675 or a mutant thereof, the *L. plantarum* CECT8678 or a mutant thereof, and the *L. plantarum* CECT8676 or a mutant thereof, wherein the strain or mutant thereof has been fermented in an artificial medium and submitted to a post-treatment after the fermentation, to obtain bacterial cells, and wherein the resulting bacterial cells are in a liquid medium or in a solid form.

In a particular embodiment, the post-treatment is selected from the group consisting of: drying, freezing, freeze-drying, fluid bed-drying, spray-drying and refrigerating in liquid medium. In a particular embodiment, the post-treatment is freeze-drying.

It is clear that by using the deposited strains as starting material, the skilled person in the art can routinely, by conventional mutagenesis or re-isolation techniques, obtain further mutants thereof that retain or enhance the herein described relevant features and advantages of the strains forming the composition of the invention. In one particular embodiment, the mutants are obtained by using recombinant DNA technology. In another embodiment, the mutants are obtained by random mutagenesis. Thus, another aspect of the disclosure relates to a method to obtain a mutant of at least one strain of *Lactobacillus plantarum,* wherein the strain of *L. plantarum* is selected from the group consisting of: CECT8675 strain, CECT8676 strain, CECT8677 strain or CECT8678 strain, wherein the method comprises using the deposited strain as starting material and applying mutagenesis, and wherein the obtained mutant further retains or enhances at least the ability of the deposited strain to antagonize the urogenital pathogen *Staphylococcus saprophyticus* as well as other UTI pathogens such as *Escherichia coli, Klebsiella pneumoniae* and/or *Proteus mirabilis.*

According to the above described beneficial features of the strains CECT8677, CECT8675, CECT8678 and/or CECT8676, another aspect of the invention relates to any of the compositions as defined above for use as a medicament or as a probiotic.

Another aspect of the invention relates to the compositions as defined above for use in the prevention and/or treatment of a condition related to alterations of the urogenital microbiota. This aspect can be alternatively formulated as the use of any of the compositions of the invention for the manufacture of a pharmaceutical product, a veterinary product, a medicament, a food product, a food supplement, a medical food, or a personal hygiene product for the prevention and/or treatment of a condition related to alterations of the urogenital microbiota. This may be also alternatively formulated as a method for the prevention and/or treatment of a condition related to alterations of the urogenital microbiota, comprising administering to the subject in need thereof an effective amount of any of the compositions of the invention. In still other particular embodiments, the condition related to alterations of the urogenital microbiota mentioned in of all the above aspects is urinary tract infection. In still other particular embodiments, the urinary tract infection is acute cystitis, acute pyelonephritis, uncomplicated UTI, complicated UTI or recurrent UTI. The compositions of the invention are particularly useful for the prophylactic treatment of individuals susceptible to UTI and also for the therapeutic management of individuals with relapsed UTI or reinfection. The compositions are also useful to prevent infections endangering the foetus in pregnant women, and preterm labour.

Alterations of the urogenital microbiota can be caused, without limitation, by hormonal changes (i.e. menstrual cycles), biomaterial-oriented contraception, sexual contact, feminine hygiene practices, antibiotic consumption, nutritional status, microbial interspecies competition, commensalism, etc. In a particular embodiment, such condition is bacterial vaginosis. Bacterial vaginosis is caused by an overgrowth of bacterial species usually absent in the vaginal flora or found in very small amounts. The most common species are *Gardnerella vaginalis* and *Atopobium vaginae.* Although bacterial vaginosis can cause unusual vaginal discharges and fishy odour, most cases are asymptomatic. A typical trait of bacterial vaginosis is the rise of the pH above 4.5, due to the disappearance of *Lactobacilli*, which further facilitates the growth of other bacterial species. Bacterial vaginosis is treated with antibiotics, such as metronidazole and clindamycin.

Another aspect of the invention relates to a pharmaceutical product, a veterinary product, a medical food, a food product, a food supplement or a personal hygiene product, comprising an effective amount of any of the compositions disclosed herein together with appropriate amounts of acceptable excipients.

Accordingly, the composition of the invention may be prepared in any suitable form which does not negatively affect the viability of the strains forming the composition of the invention. Selection of the excipients and the most appropriate methods for formulation in view of the particular purpose of the composition is within the scope of ordinary persons skilled in the art of pharmaceutical technology.

The composition can be administered orally, vaginally or rectally, or using different administration forms simultaneously (e.g. orally and vaginally).

In this sense, the composition of the invention is in solid or liquid form and may be, *inter alia*, in the form of powders, tablets or lozenges, sucking tablets, film preparations, solutions, aerosols, granules, pills, suspensions, emulsions, capsules, enterocoated tablets and capsules, syrups, liquids, elixirs, sweets, chewing gum, suppositories, micro-enemas, vaginal tablets, vaginal gelatine capsules, creams, gels, ointments, lotions, tampons, napkins, pads, melting strips, condoms, pessaries, sprays, douches, tincture or fluid extracts.

In a particular embodiment, the composition of the invention is administered in combination with other active ingredients commonly used to treat UTI, in particular with an antimicrobial agent. In previous sections, it is defined what is understood as antimicrobial agents, commonly including antibiotics and natural products and ingredients with antimicrobial effect. Particular embodiments of the invention are compositions comprising the strains of the invention together with: cranberry; cranberry and salvia; cranberry, mannose and salvia; cranberry and vitamin C; cranberry, berberine, arbutin and betula; and cranberry, berberine, arbutin, betula and mannose.

The composition according to the invention can be formulated in a form in which the strains of the invention are the only active agent. Alternatively, the composition of the invention can be formulated mixed with one or more other active agents. Alternatively, the strains of the invention either alone or formulated mixed with one or more other active agents, can also be formulated with pharmaceutically or veterinary acceptable excipients or adequate additives or ingredients in the case of a food product. In a particular embodiment of the invention, the composition additionally contains one or more further active agents. Alternatively, the additional active agent or agents are other probiotic bacteria which are not antagonistic to the strains forming the composition of the invention. Particularly, and due to the capacity of the strains of the invention to survive in high concentrations of antibiotic agents to which the strains of the invention are resistant and which are typically used to manage UTI, the composition of the invention can be administered in combination with fosfomycin in the prevention and/or treatment of UTI. As shown in EXAMPLE 5, advantageously, the strains of the invention can be co-administrated with fosfomycin, which is first-line treatment in many countries. Since resistance of uropathogens (e.g. S. *saprophyticus*) to this antibiotic has significantly increased in the recent years in many countries, the combined use of the probiotic strain(s) of the invention with fosfomycin can increase the inhibitory spectrum of this antibiotic, thus improving its efficacy.

Thus, in a particular embodiment, the compositions of the invention are for use in combination with an effective amount of an antimicrobial agent; and more particularly, the antibiotic is fosmomycin.

In a particular embodiment, the composition comprises more than one active agent, the active agents may be formulated in a single pharmaceutical, veterinary, food supplement or medical food forms, or in separate forms to be administered sequential or consecutively in any order, wherein the form comprising at least one of the strains of the present invention, is administered prior to, substantially simultaneously, after or in between the administration of the other active agent(s).

Daily dosages and therapeutic regimens will be determined by the skilled in their art. For example, daily dosage may be higher during the treatment of active symptoms or may be lower for prophylactic purposes. A preferred therapeutic regimen will be based on the simultaneous administration of the lactic acid bacteria and the antimicrobial agents or other active ingredients. However, therapeutic regimens can be also sequential, e.g. administrating first the antimicrobial agent or active ingredient of interest and starting the treatment with the probiotic composition according to the present invention some days later. Other typical therapeutic regimens consist on starting the treatment with probiotic composition prior to the administration of the antimicrobial agent or active ingredient of interest.

Additionally, the administration can be chronic or intermittent, as deemed appropriate by the supervising practitioner, particularly in view of any change in the disease state or any undesirable side effects. "Chronic" administration refers to administration of the composition in a continuous manner while "intermittent" administration refers to treatment that is done with interruption.

The effective amount of colony forming units (cfu) for the strains in the composition will be determined by the skilled in the art and will depend upon the final formulation. For instance, when administered orally without any other active agent, the total amount of the strains of the invention is present in the composition in a single dose in amount giving an effective daily dose of from 10⁷ to 10¹² cfu, according to the current legislation, preferably from 10⁹ to 10¹¹ cfu and, when administered vaginally or rectally, in an amount giving an effective daily dose of from 10³ to 10¹² cfu, preferably from 10⁵ to 10¹⁰ cfu. The term "colony forming unit" ("cfu") is defined as number of bacterial cells as revealed by microbiological counts on agar plates. Food supplements usually contain probiotic strains in an amount ranging from 10⁷ and 10¹² cfu/g. In a particular embodiment, the composition of the invention is a food supplement for daily doses comprising between 10⁹-10¹¹ cfu/g.

The term "pharmaceutical product" is understood in its widely meaning in this description, including any composition that comprises an active ingredient, in this case, the strains of the invention preferably in form of composition, together with pharmaceutically acceptable excipients. The term "pharmaceutical product" is not limited to medicaments. The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc. must also be "acceptable" in the sense of being compatible with the other ingredients of the formulation. Suitable carriers, excipients, etc. can be found in standard pharmaceutical texts.

The term "excipient" is understood in its widely meaning in this description, including any natural or synthetic substance formulated alongside the active ingredient of a pharmaceutical product, veterinary product, a medicament, food supplement, medical food and personal hygiene product. The most appropriate excipient(s) to be used depend(s) of the final formulation.

Excipients are selected, without limitation, from the group comprising: fillers/diluents/bulking agents, binders, antiadherents, disintegrants, coatings, anti-caking agents, antioxidants, lubricants, sweeteners, flavors, colours, tensides and other classes of pharmaceutically and veterinary acceptable excipients.

Fillers are selected, without limitation, from the group comprising: inulin, oligofructose, pectin, modified pectins, microcrystalline cellulose, lactose, starch, maltodextrin, saccharose, glucose, fructose, mannitol, xylitol, non-crystallizing sorbitol, calcium carbonate, dicalcium phosphate, other inert inorganic and organic pharmacologically acceptable fillers, and mixtures of these substances. At dosage form of oral suspension, fillers or diluents are selected from the group comprising: vegetable oil, oleic acid, oleyl alcohol, liquid polyethylene glycol, other pharmacologically acceptable inert liquids, or mixtures of these substances.

Binders are used in solid dosage forms, e.g. to hold the ingredients in a tablet together, to ensure that tablets and granules can be formed with required mechanical strength, and to give volume to low active dose tablets. Binders in solid dosage forms like tablets are: lactose, sucrose, corn (maize) starch, modified starches, microcrystalline cellulose, modified cellulose (for example hydroxypropyl methylcellulose (HPMC) and hydroxyethylcellulose), other water soluble cellulose ethers, polyvinylpyrrolidone (PVP) also known as povidone, polyethylene glycol, sorbitol, maltitol, xylitol and dibasic calcium phosphate; other suitable pharmacologically acceptable binders, or mixtures of these substances.

Antiadherents are used to reduce the adhesion between the powder (granules) and the punch faces and thus prevent sticking to tablet punches. They are also used to help protect tablets from sticking. The most commonly used is magnesium stearate.

As disintegrants and superdisintegrants in solid dosage forms like tablets and capsules, the following substances, without limitation, are used: cross-linked polyvinylpyrrolidone, sodium starch glycolate, sodium carboxymethyl cellulose, calcium carboxymethyl cellulose, and formaldehyde-casein, other suitable pharmacologically acceptable disintegrant and superdisintegrant, or their mixtures.

Coatings in the case of solid dosage forms, such as tablets and granules for capsules filling, protect the ingredients from deterioration by moisture in the air, make large, unpleasant-tasting tablets easier to swallow and/or in the case of enteric coatings ensure intact passage through a strong acidic medium of gastric juice (pH around 1), and which allow release in duodenum or ileum (small intestine). For most coated tablets, a cellulose ether hydroxypropyl methylcellulose (HPMC) film coating is used. Occasionally, other coating materials are used, for example synthetic polymers and co-polymers like polyvinylacetate phthalate (PVAP); co-polymers of methyl acrylate-metacrylic acid; co-polymers of methyl metacrylate-metacrylic acid; shellac, corn protein zein or other polysaccharides; waxes or wax-like substances such as beeswax, stearic acid; higher fatty alcohols like cetyl or stearyl alcohol; solid paraffin; glycerol monostearate; glycerol distearate, or their combinations. Capsules are coated with gelatin or hydroxypropyl methylcellulose.

Enteric coatings control the rate of drug release and determine where the drug will be released in the digestive tract. Materials used for enteric coatings include fatty acids, waxes, shellac, plastics, and plant fibers and their mixtures, also in combination with other above mentioned coatings.

An anticaking agent is an additive placed in powdered or granulated materials to prevent the formation of lumps (caking) and for easing packaging, transport, and consumption. As anti-caking agents in solid dosage forms like tablets, capsules, or powders, the following are used: magnesium stearate, colloidal silicon dioxide, talc, other pharmacologically acceptable anticaking agents, or their mixtures.

Lubricants are used in solid dosage forms, in particular in tablets and capsules, to prevent ingredients from clumping together and from sticking to the tablet punches or capsule filling machine, and also in hard capsules. As lubricants talc or silica, and fats, e.g. vegetable stearin, magnesium stearate or stearic acid, and mixtures thereof, are the most frequently used lubricants in tablets or hard gelatin capsules.

Sweeteners are added to make the ingredients more palatable, especially in solid dosage forms, e.g. chewable tablets, as well as in liquids dosage forms, like cough syrup. Sweeteners may be selected from artificial, natural or synthetic or semi-synthetic sweeteners; non-limiting examples of sweeteners are aspartame, acesulfame potassium, cyclamate, sucralose, saccharine, sugars or any mixture thereof,

Flavors can be used to mask unpleasant tasting active ingredients in any dosage form. Flavorings may be natural (e.g. fruit extract) or artificial. For example, to improve: (1) a bitter product, mint, cherry or anise may be used; (2) a salty product, peach or apricot or liquorice may be used; (3) a sour product, raspberry; and (4) an excessively sweet product, vanilla.

Except auxiliary substances from the class of excipients, the formulation from the present invention can contain other pharmacologically active or nutritive substances selected from the group comprising: antibiotics; cranberry juice; vitamins, such as vitamin C (or ascorbic acid) in the pharmaceutically acceptable chemical form, salt or derivatives; minerals in the form of pharmaceutically and nutritive acceptable chemical form; and L-amino acids.Regarding the preparation of the formulations of the present invention is within the scope of ordinary person skilled in the art and will depend upon the final dosage formulation. For instance, and without limitation, when the final dosage forms is an oral solid one, such as tablets, capsules, powder, granules, oral suspension, etc. the process for preparation of solid dosage forms of the formulation includes homogenization of: (1) the active ingredient(s), comprising at least one or more post-treated probiotic bacteria of the invention in an effective amount; (2) with one or more excipients to form homogeneous mixture which is, e.g. according to requirements, subjected to lubrication with magnesium stearate or other lubricants yielding final dosage form of powder. Such homogeneous powder is filled into ordinary gelatin capsules or, alternatively, into gastro-resistant capsules. In the case of tablets, they are manufactured by direct compression or granulation. In the first case, a homogeneous mixture of active ingredients and suitable excipients such as anhydrous lactose, non-crystallizing sorbitol, and others is prepared. In the second case, tablets are processed on the mixture in granulated form. Granules are prepared by granulation process of active ingredients of the formulation with suitable fillers, binders, disintegrants, and small amount of purified water. Such prepared granules are sieved and dried until the water content of <1 % w/w.

Regarding the process for preparation of liquid dosage forms (e.g. oral suspension), it involves homogenization of the active ingredient(s) of the formulation comprising at least one or more post-treated probiotic bacteria of the invention in an effective amount in an inert liquid diluent (filler) such as various vegetable oils like sunflower, soybean or olive oil; oleic acid; oleyl alcohol; liquid polyethylene glycols like PEG 200, PEG 400 or PEG 600; or other inert pharmacologically acceptable liquids. The process further involves treatment of homogeneous mixture with one or more processes selected from the group comprising: (1) stabilization of the formulation, by addition and homogenization of suspension stabilizers like beeswax, colloidal silicon dioxide, etc.; (2) sweetening of the formulation; by addition and homogenization of sweetener; (3) flavoring of the formulation, by addition and homogenization of flavoring. Such forms of the formulation can contain also other excipients or ingredients, usually employed in the art.

The pharmaceutical product can adopt different forms or names depending on the product approval route and also depending on the country. For instance, a medicament is a particular pharmaceutical product. A medical food is another particular pharmaceutical product. The terms "medical food" or "food for special medical purposes" are used in some countries to refer to a food specially formulated and intended for the dietary management of a disease that has distinctive nutritional needs that cannot be met by normal diet alone. They are defined in regulations such as the Food and Drug Administration's 1988 Orphan Drug Act Amendments in the United States, and the Commission Directive 1999/21/EC in Europe. Medical foods are distinct from the broader category of food supplements and from traditional foods that bear a health claim. Thus, in a particular embodiment, the composition of the invention is a medical food.

Often, probiotic bacterial compositions such as the one disclosed herein, are considered as food supplements. A food supplement, also known as dietary supplement or nutritional supplement is considered another particular pharmaceutical product. This is a preparation or product intended to supplement the diet, made from compounds usually used in foodstuffs, which provide nutrients or beneficial ingredients that are not usually ingested in the normal diet or may not be consumed in sufficient quantities. Mostly, food supplements are considered as food products, but sometimes they are defined as drugs, natural health products, or nutraceutical products. In the sense of the present invention, food supplements also include nutraceuticals. Food supplements are usually sold "over the counter", i.e. without prescription. If the food supplement adopts the form of a pill, a capsule a tablet or a powder, it comprises excipients which are the same as the used in medicaments. A food supplement however, can also adopt the form of a food product which is fortified with some nutrients (e.g. a bar or yoghurt). Thus, in a particular embodiment, the composition of the invention is a food supplement.

If the composition according to the invention is a food supplement, it can be administered as such, can be mixed with a suitable drinkable liquid, such as water, yoghurt, milk or fruit juice, or can be mixed with solid or liquid food. In this context the food supplement can be in the form of tablets or lozenges, pills, capsules, granules, powders, suspensions, sachets, sweets, bars, syrups and corresponding administration forms, usually in the form of a unit dose. Preferably, the food supplement comprising the composition of the invention is administered in the form of tablets, lozenges, capsules or powders, manufactured in conventional processes of preparing dietary supplements.

The strains of the invention can be also included in a variety of food products, such as a milk products (a yogurt, a cheese, a fermented milk, a milk powder, a milk based fermented product, an ice-cream, a fermented cereal based product, a milk based powder), bread, bars, spreads, biscuits and cereals, a beverage, different types of oil, or a dressing. The term "food product" is used herein in its broadest meaning, including any type of product, in any form of presentation, which can be ingested by an animal, but excluding pharmaceutical and veterinary products. Examples of other food products are meat products (e.g. sausages or meat spreads), chocolate spreads, fillings and frostings, chocolate, confectionery, baked goods (cakes, pastries), sauces and soups, fruit juices and coffee whiteners. Particularly interesting food products are food supplements and infant formulas. The food product preferably comprises a carrier material such as oat meal gruel, lactic acid fermented foods, resistant starch, dietary fibres, carbohydrates, proteins and glycosylated proteins. In a particular embodiment the strains of the invention are encapsulated or coated. Hence, in a particular embodiment, the composition of the invention is a food product.

Some of the aforementioned forms are considered medical devices in some countries; e.g. vaginal capsules, a tampon or other types of applicators. Thus, in a particular embodiment, the composition of the invention is a medical device.

In another particular embodiment, the composition of the invention is a personal hygiene product, which can be sold "over the counter" in a supermarket or in a pharmacy. Examples of personal hygiene product are tampons, sanitary napkins, sanitary pads, diapers, soaps, shampoos, gels, ointments, creams, sprays and lotions.

Particularly, the composition of the invention is in the form of disintegrable mucoadhesive intravaginal tablets which are applied intravaginally using an applicator device.

Thus, it has to be understood that the composition of the invention is useful in the management of UTI and other related conditions of the urogenital tract regardless of the form of the composition; i.e. regardless of being a pharmaceutical product, a medicament, a food product, a food supplement, a medical food, or a personal hygiene product.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided herein for illustrative purposes, and without intending to be limiting to the present invention.

### BRIEF DESCRIPTION OF THE DRAWINGS

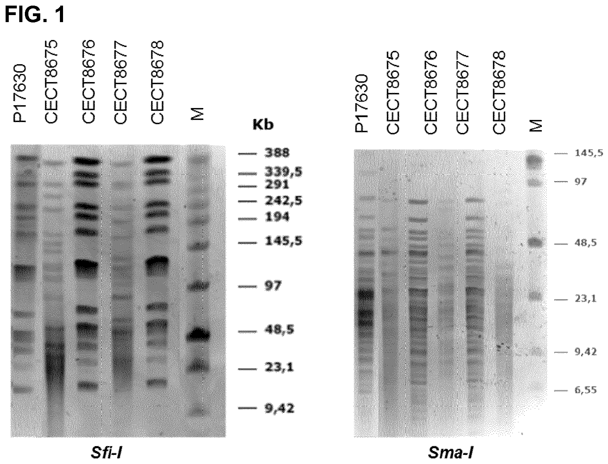

FIG. 1. Pulsed-field electrophoresis patterns of *Sfi-*I and *Sma-*I restricted genomic DNA of (from left to right): *Lactobacillus plantarum* P17630, *L. plantarum* CECT8675, *L. plantarum* CECT8676, *L. plantarum* CECT8677, *L. plantarum* CECT8678, and DNA molecular marker (M).

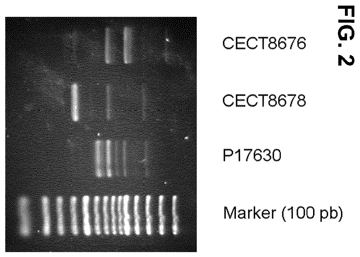

FIG. 2. RAPDs of (from left to right): *L. plantarum* CECT8676, *L. plantarum* CECT8678, the commercial probiotic strain *L. plantarum* P17630, and DNA molecular marker (Marker).

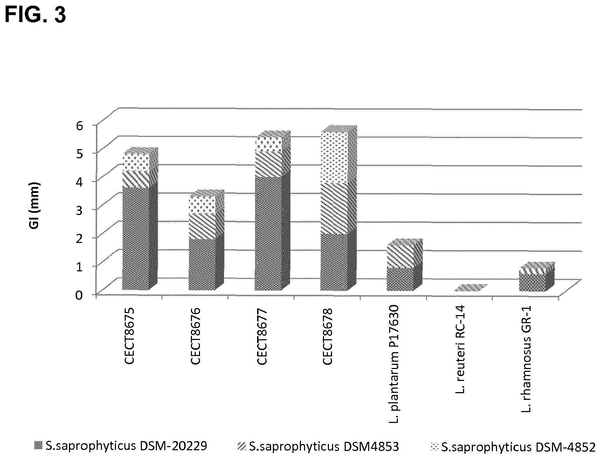

FIG. 3. Stacked Column Bar Chart showing the antagonistic accumulative activity of *L. plantarum* CECT8675, CECT8676, CECT8677, CECT8678 strains and the commercial strains *L. plantarum* P17630, *L. reuteri* RC-14® and *L. rhamnosus* GR-1®, which were used as controls, against 3 different strains of *Staphylococcus saprophyticus.* This bar chart provides a visual presentation showed as separate stacked bar/column. Each stacked bar/column results from grouping the GI or inhibitory activity ("GI (mm)") measured in millimetres for each of the above cited strains as well as for each mentioned control strains, against DSM20229, DSM4853 and DSM4852 strains of *S. saprophyticus.* In this chart the strain tested for the antagonistic activity against *S. saprophyticus* appears along the horizontal axis; and the height of the stacked bar corresponds to the accumulative GI value of each tested strain. Accordingly, each stacked bar represents the accumulative GI activity of each tested strain against DSM20229, DSM4853 and DSM4852 strains of S. *saprophyticus* accumulated on top of each other. The height of the resulting stacked bar shows the combined result.

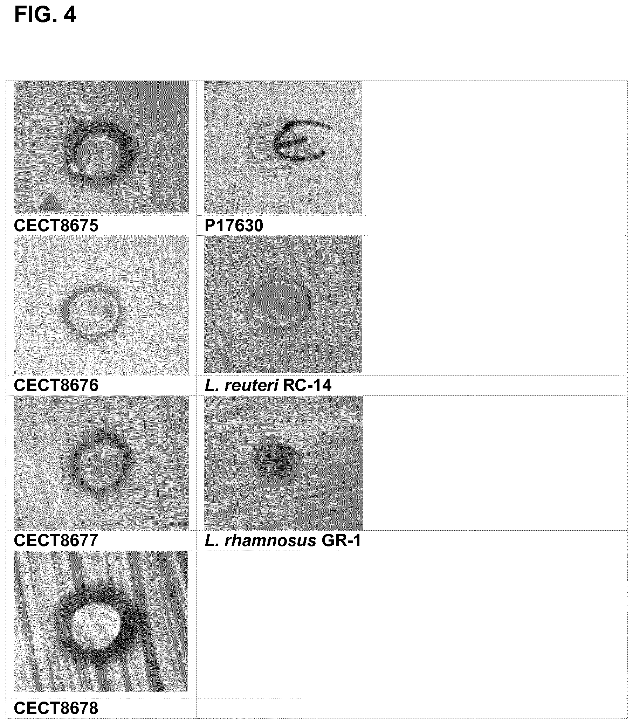

FIG. 4 shows the halos (also known as plaques or zones of inhibition) of *L. plantarum* CECT8675, CECT8676, CECT8677 and CECT8678 strains of the invention and the commercial strains *L. plantarum* P17630. *L. reuteri* RC-14® and *L. rhamnosus* GR-1® were used as controls against the pathogenic *Staphylococcus saprophyticus* DSM4582. As depicted in FIG. 4 the larger sizes of the halos, representing the clear zones or zones of inhibition of growth of the pathogen S. *saprophyticus* DSM4582, were obtained for the strains of the invention compared with the control strains, when placed on a plaque of S. *saprophyticus* DSM4582 grown to confluence. This means more potent antagonistic activity of the strains of the invention compared to the commercial used strains.

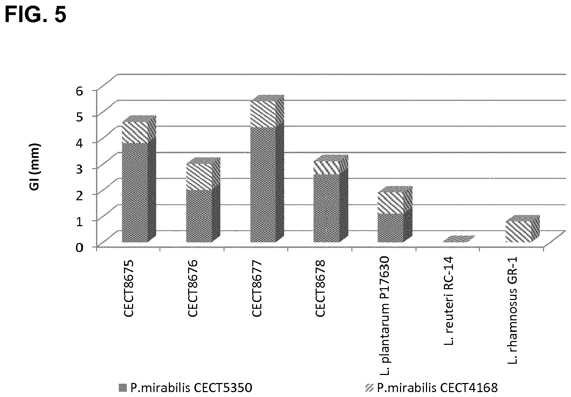

FIG. 5. Stacked Column Bar Chart showing the antagonistic accumulative activity of *L. plantarum* CECT8675, CECT8676, CECT8677, CECT8678 strains, and the commercial strains *L. plantarum* P17630, *L. reuteri* RC-14® and *L. rhamnosus* GR-1®, which were used as controls against 2 different strains of *Proteus mirabilis.* Each stacked bar/column results from grouping the GI or inhibitory activity ("GI (mm)") measured in millimetres for each of the above cited strains as well as for each tested control strain, against CECT5350 and CECT4168 strains of *P. mirabilis.* The strain tested for the antagonistic activity against *P. mirabilis* appears along the horizontal axis; and the height of the stacked bar corresponds to the accumulative GI value of each tested strain. Accordingly, each stacked bar represents the accumulative GI activity of each tested strain against CECT5350 and CECT4168 strains of *P. mirabilis* accumulated on top of each other. The height of the resulting stacked bar shows the combined result.

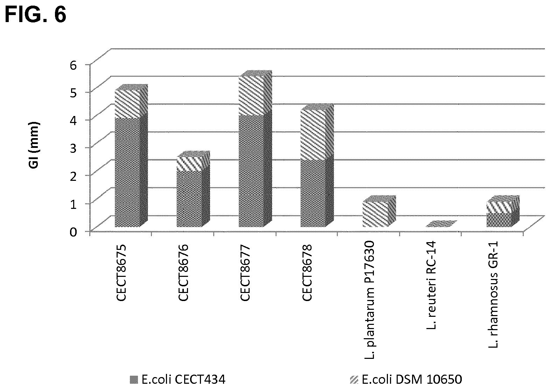

FIG. 6. Stacked Column Bar Chart showing the antagonistic accumulative activity of *L. plantarum* CECT8675, CECT8676, CECT8677, CECT8678 strains, and the commercial strains *L. plantarum* P17630 *L. reuteri* RC-14® and *L. rhamnosus* GR-1® which were used as controls against 2 different strains of *E. coli.* Each stacked bar/column results from grouping the GI or inhibitory activity ("GI (mm)") measured in millimetres for each of the above cited probiotic strains as well as for each tested control strain, against CECT434 and DSM10650 strains of *E. coli.* In this chart the strain tested for the antagonistic activity against *E. coli* appears along the horizontal axis; and the height of the stacked bar corresponds to the accumulative GI value of each tested strain. Accordingly, each stacked bar represents the accumulative GI activity of each tested strain against CECT434 and DSM10650 strains of *E. coli* accumulated on top of each other.

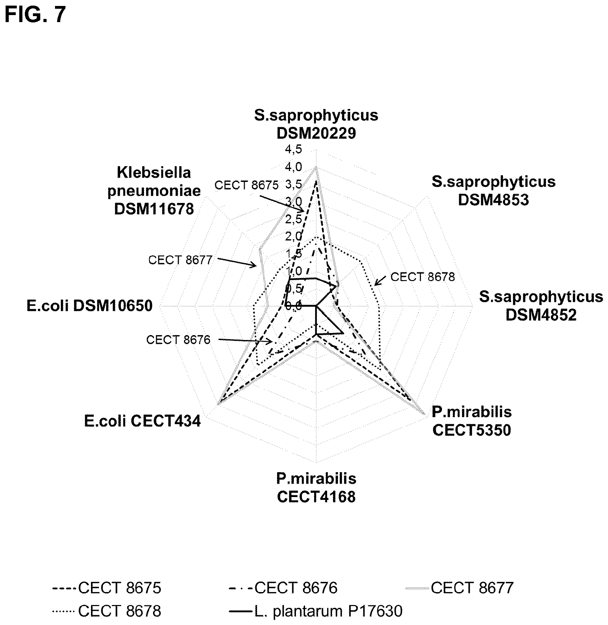

FIG. 7 shows the overall antagonistic activity against most relevant UTI pathogens. The strains of the invention show a higher general antagonistic spectrum activity.

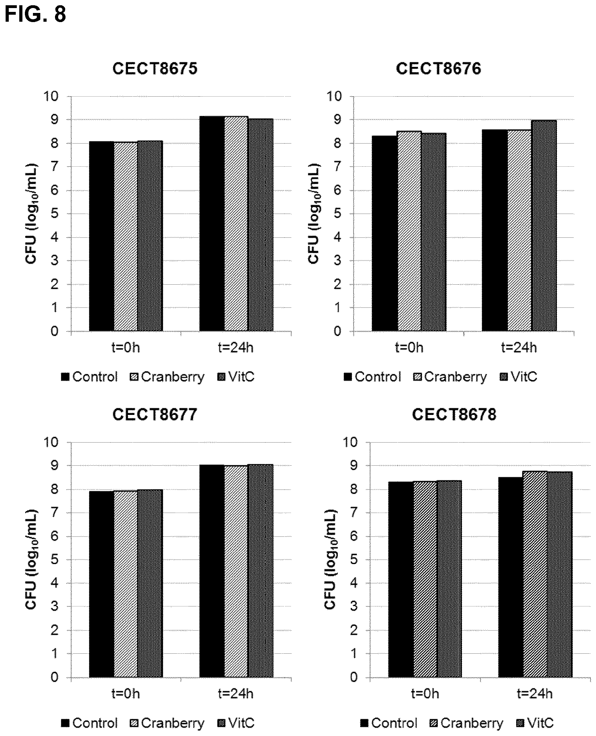

FIG. 8. Column Bar Charts for strains CECT8675, CECT8676, CECT8677, CECT8678 showing the compatibility of each strain with cranberry extract and with vitamine C, active ingredients commonly used for formulating products for treating UTI in an *in vitro* study.
FIG. 9. Combined use of antibiotic (fosfomycin) and probiotic (*L*. *plantarum* CECT8677) against *Staphylococcus saprophyticus.* Results expressed as log₁₀ colony formation units (CFU) per mL of S. *saprophyticus* when incubated A) without antibiotic or probiotic (positive control), B) with antibiotic (fosfomycin) or C) with antibiotic (fosfomycin) and probiotic (*L. plantarum* CECT8677).

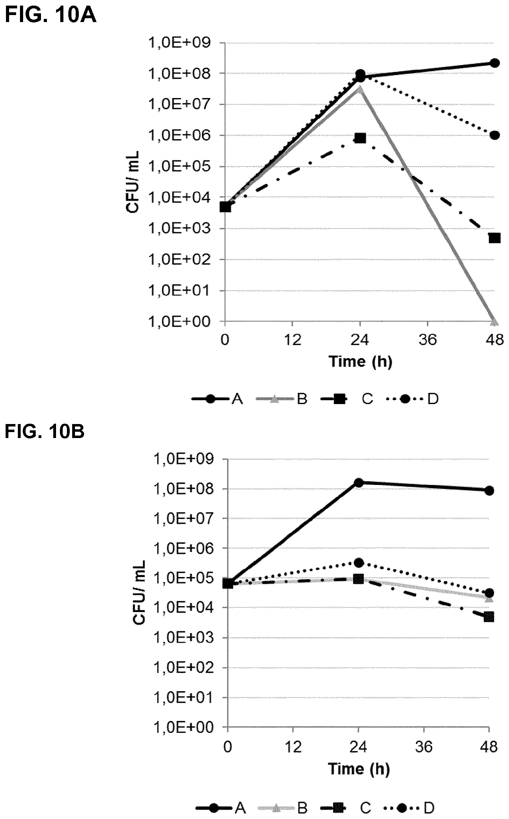

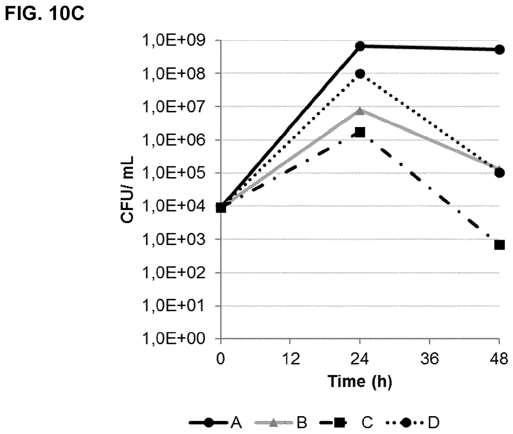

FIG. 10. Growth of UTI pathogens in the presence or absence of lactobacilli strains. 10A, growth kinetics of *Staphyloccoccus saprophyticus*; 10B, growth kinetics of *Escherichia coli*; 10C, growth kinetics of *Proteus mirabillis.* Growth kinetics of pathogens were studied A) in the absence of probiotic as control, B) in the presence of *L. plantarum* CECT8675, C) in the presence of *L. plantarum* CECT8677 or D) in the presence of *L. rhamnosus* GR-1

### EXAMPLES

### EXAMPLE 1. Isolation of the microorganisms

Strains CECT8675, CECT8676, CECT8677 and CECT8678 were isolated from fresh stools of 0-9 year-old children. Faeces were dissolved in PBS buffer (pH 7.4), aliquoted and plated on MRS supplemented with various antibiotic combinations. Strains were cultured under microaerophilic conditions (5 % CO₂) at 37 or 30 °C. Incubation time depended on the growth rate, but ran normally from 24 hours to 3 days. Isolation of individual strains proceeded with the same selection media, and then Gram staining was carried out in order to get a first identification. Once grown, isolated strains were stored by freeze-drying in PBS 0.1x with 15 % skim milk powder.

The strains were initially grown on MRS medium supplemented with 10 µg/ml vancomycin (Sigma Chemical Co, Germany) and incubated at 30 °C under anaerobic conditions and pH 6.4. Gram staining showed a clear Gram positive staining, as well as non-spore-forming bacilli morphology.

### EXAMPLE 2. Genus and species identification

Identification to species level was performed by sequencing 16S rRNA gene. Briefly, DNA of the strains was extracted with Chelex® 100 resin from Bio-Rad Laboratories (Barcelona, Spain). Complete sequence of 16S rRNA gene was amplified by polymerase chain reaction (PCR) using the universal primers for eubacteria 27F and 1492R as previously described [LANE, D.J. 16/23S rRNA sequencing. In Nucleic acid techniques in bacterial systematics (ed. Stackebrandt and Goodfellow) 1991, pages 177-204; MUYZER, G. et al. Application of denaturing gradient gel electrophoresis (DGGE) and temperature gradient gel electrophoresis (TGGE) in microbial ecology. Antonie van Leeuwenhoeck. 1998. Vol 73, No 1, pages 127-141]. The integrity of PCR products was checked in an agarose gel using SYBR green dye (Invitrogen, Life Technologies, Madrid, Spain). PCR products were sequenced using 27F, 357F, 907R and 1492 primers [LANE, D.J. *supra*; MUYZER; G. *et al. supra*; OLSEN, Q.J et al. Microbial Ecology and Evolution: A Ribosomal RNA Approach. Annual Review of Microbiology. 1986. Vol. 4, No. 1, pages 337- 365), a v3.1 Cycle Sequencing kit and a 3130 XL Genetic Analyzer (from Applied Biosystems, Life Technologies, Madrid, Spain). The resulting sequences were aligned and compared with those presents in the National Center for Biotechnology Information (NCBI) and RDP (Ribosomal Database Project). The strains were identified based on the highest hit scores.

Strains CECT8675, CECT8676, CECT8677 and CECT8678 were identified as *Lactobacillus plantarum.* They were deposited in the Spanish Type Culture Collection (CECT) under the accession numbers CECT8675, CECT8676, CECT8677 and CECT8678.

The 16S rRNA sequences obtained for strains CECT8675, CECT8676, CECT8677 and CECT8678, correspond to SEQ ID NO: 1, SEQ ID NO: 2, SEQ ID NO: 3 and SEQ ID NO: 4, respectively.

### EXAMPLE 3. Strain genotyping

Strain genotyping was performed by genomic digestion and pulsed field gel electrophoresis (PFGE), and random amplified polymorphic DNA (RAPD). PFGE of the strains was performed as previously described [RODAS, A.M. et al. Polyphasic study of wine Lactobacillus strains: taxonomic implications. Int. J. Syst. Evol. Microbiol, 2005. Vol. 55, No. 1, pages 197-207) using Sfi-I and Sma-I restriction enzymes. RAPD was performed as described by Nigatu *et al.* (NIGATU, A. et al. Randomly amplified polymorphic DNA (RAPD) for discrimination of Pediococcus pentosaceus and Pediococcus acidilactici and rapid grouping of Pediococcus isolates. Letters in Applied Microbiology. 1998. Vol 26, No. 6, pages 412-6). Patterns were compared with *L. plantarum* P17630 from Isadin α Barcilus® product as a commercially available strain from the same species.

PFGE patterns are depicted in FIG.1. It can be clearly seen that the pattern of strains CECT8675 and CECT8677 were different from those of the other strains; whereas the restriction pattern of strains CECT8676, CECT8678 and P17630 were very similar between them. Therefore, RAPD was performed as a complementary method for distinguishing strains CECT8676, CECT8678 and P17630, because it is an efficient technique for genotyping *Lactobacillus plantarum* strains (see for instance JOHANSSON, M.L. et al. Randomly amplified polymorphic DNA (RAPD) for rapid typing of Lactobacillus plantarum strains. Appl. Microbiol. 1995. Vol. 21, No. 3, pages 155-159). Results are shown in FIG. 2 and allow to clearly confirming that strains CECT8676, CECT8678 and P17630 were also different.

### EXAMPLE 4. Antagonistic activity against most frequent pathogens causing UTI

The capacity of the probiotic strains of the present invention to inhibit the growth of the most frequent pathogens causing UTI was studied by confronting pathogen seed plates with cylinder sections of the probiotic strains. In particular, different strains of the following pathogens were analysed: *Staphylococcus saprohyticus, Proteus mirabilis, Escherichia coli* and *Klebsiella pneumoniae.*

Probiotic strains were uniformly seeded in MRS agar plates and allowed to grow to confluence for 24h at 37 °C and 5 % of CO₂. Pathogen strains were cultured overnight in TSA (Trypticase Soy Agar) plates at 37 °C and 5 % of CO₂. Isolated colonies of these pathogens were used to prepare suspensions in phosphate buffered saline (PBS) medium and swabbed uniformly in TSA plates. Immediately cylindrical sections of 6 mm in diameter of the confluent agar plate of the tested probiotic strains were placed lane-to-lane on to the pathogen seeded plate, confronting the pathogen seeded plate with the grown-agar side of one of the cylinder sections and with the non-grown side of the other cylinder section and incubated overnight at 37 °C and 5 % of CO₂. Then, inhibition zones were measured by placing the agar plate over a flat rule. Growth inhibitory activity (GI) was then calculated by subtracting the cylinder diameter (CD) from the inhibition zone diameter (IZD) measured in millimetres and dividing this difference by 2, following the formula GI = (IZD-CD) / 2. The final inhibitory activity was calculated as a mean value of the GI values for the two above-mentioned cylinder sections for each probiotic strain, i.e. averaging the duplicates. Values lower than 0.5 mm were considered as no inhibition (n.i.). All experiments were performed in triplicate.

The results are detailed in the following sections 4.1-4.3 and in Tables 1-3. For illustrative purposes the following codes of activity were used in said Tables:"-" means GI<0.5 (=no quantifiable inhibition); "+" means 0.5≤GI<1; "++" means 1≤GI<2; "+++" means 2≤GI<3; and "++++" means GI≥3. Finally, "n.r." stands for "Not reported value".

Accordingly, Isadin α Barcilus® has been used as baseline for comparing the probiotic performance of *Lactobacillus* used for restoring microbial balance of the vaginal ecosystem. As shown in TABLES 1-3, the orally administered probiotic strain *L. plantarum* P17630 of Isadin α Barcilus® which has been shown to be effective in the restoration of the vaginal lactobacilli flora and reduction of its colonization by potentially pathogenic bacteria, is not effective in reducing the recurrence of UTI, in view of the lack of antagonistic activity against UTI pathogenic agents, *S. saprophyticus, P. mirabilis* and *E. coli.* Hence, a commercial probiotic composed of a *Lactobacillus* strain, in particular, *L. plantarum* P 17630 strain, in other words a lactobacillus from the same species as that of the strains of the present invention has not disclosed antagonistic effect against pathogens causative of UTI. Finally, the commercial probiotic food VSL#3 for the dietary management of patients with ulcerative colitis, IBS, etc., comprising also lactobacilli strains have neither disclosed the antagonistic activity of the strains of the present invention.

### 4.1. Antagonistic activity against Staphylococcus saprophyticus

Lactobacilli strains were tested against three strains of *S. saprophyticus* (CECT235, DSM4853 and DSM4852).

As shown in TABLE 1, all strains CECT8675, CECT8676, CECT8677 and CECT8678 of the present invention showed antagonistic activity against the three tested strains of *S. saprophyticus.* In contrast, the commercial strain *L. reuteri* RC-14® showed no inhibition against any of the three tested strains of *S. saprophyticus. L. rhamnosus* GR-1® was able to inhibit the growth of only one of the three tested strains of *S. saprophyticus*; and commercial strains *L. plantarum* P17630 and VSL#3 were unable to inhibit one of the three strains of *S. saprophyticus.*

**TABLE 1**

| Strain | *S. saprophyticus* CECT235 | *S. saprophyticus* DSM4853 | *S. saprophyticus* DSM4852 |
|---|---|---|---|
| CECT8675 | **++++** | **+** | **+** |
| CECT8676 | **++** | **+** | **+** |
| CECT8677 | **++++** | **+** | **+** |
| CECT8678 | **+++** | **++** | **++** |
| P17630 | **+** | **+** | **-** |
| *L. rhamnosus* GR-1® | **+** | **-** | **-** |
| *L. reuteri* RC-14® | **-** | **-** | **-** |
| *L. plantarum* VSL#3 | **-** | **n.r.** | **n.r.** |

FIG. 3 shows the antagonistic accumulative activity against strains of S. *saprophyticus* of the strains of the invention and of the commercial probiotic strains *L. plantarum* P17630, *L. reuteri* RC-14® and *L. rhamnosus* GR-1®. All four strains of the invention showed higher activity against *S. saprophyticus* when compared with the commercial strains.

FIG. 4 shows the halos (also known as plaques or zones of inhibition) against *S. saprophyticus* of both, the strains of the invention and the commercial strains used as controls. As depicted in FIG. 4 the larger sizes of the halos obtained for the strains of the invention, the more potent antagonistic activity of the strains of the invention compared to the commercial strains used to compare with.

### 4.2. Antagonistic activity against Proteus mirabilis

Lactobacilli strains were tested against two strains of *P. mirabilis* (CECT5350 and CECT4168).

As shown in TABLE 2 below, all the tested strains of the present invention showed antagonistic activity against the two tested strains of *P. mirabilis.* This was also the case of *L. plantarum* P17630. However, as depicted in FIG. 5, the accumulative antagonistic activity of the strains of the invention was higher than that of P17630. The commercial strain *L. reuteri* RC-14® showed no antagonistic activity.

**TABLE 2**

| Strain | *P. mirabilis* CECT5350 | *P. mirabilis* CECT4168 |
|---|---|---|
| CECT8675 | **++++** | **+** |
| CECT8676 | **+++** | **++** |
| CECT8677 | **++++** | **++** |
| CECT8678 | **+++** | **+** |
| P17630 | **++** | **+** |
| *L. rhamnosus* GR-1® | **-** | **+** |
| *L. reuteri* RC-14® | **-** | **-** |
| *L. plantarum* VSL#3 | **+** | **n.r.** |

### 4.3. Antagonistic activity against Escherichia coli and Klebsiella pneumoniae

Lactobacilli strains were tested against two strains of *E. coli* (CECT434 and DSM10650) and *K. pneumonia* DSM11678 strain.

As shown in TABLE 3, all the strains of the present invention showed antagonistic activity against the two strains of *E. coli* tested. This was not the case of the other commercial strains commonly used for treating vaginal conditions, which inhibited one or none of the tested *E. coli* strains. This demonstrates that although these commercial strains can be useful for treating conditions such as vaginitis (*L. plantarum* P17630) and vaginosis (*L. rhamnosus* GR-1® and *L. reuteri* RC-14®), they are not necessarily effective for treating UTI. FIG. 6 depicts the higher antagonistic accumulative activity against the two strains of *E. coli* of the strains of the invention compared with the commercial strains.

Advantageously, all the strains of the present invention also showed antagonistic activity against the strain of *K. pneumoniae* tested, which was not the case of *L. rhamnosus* GR-1® and *L. reuteri* RC-14®.

**TABLE 3**

| Strain | *E. coli* CECT434 | *E. coli* DSM10650 | *K. pneumoniae* DSM11678 |
|---|---|---|---|
| CECT8675 | **++++** | **++** | **++** |
| CECT8676 | **+++** | **+** | **+** |
| CECT8677 | **++++** | **++** | **+++** |
| CECT8678 | **+++** | **++** | **++** |
| P17630 | **-** | **+** | **++** |
| *L. rhamnosus* GR-1® | **+** | **-** | **-** |
| *L. reuteri* RC-14® | **-** | **-** | **-** |
| *L. plantarum* VSL#3 | **-** | **n.r.** | **n.r.** |

### EXAMPLE 5. Compatibility of the strains with antibiotic treatment for UTI

The capacity of strains CECT8675, CECT8676, CECT8677 and CECT8678 to growth in the presence of fosfomycin, which is used as a first-line antibiotic therapy for treating UTI, was evaluated *in vitro.* The minimal inhibitory concentration (MIC) of fosfomycin was studied for said strains according to ISO 10932:2010 (IDF 223:2010) Milk and milk products - Determination of the minimal inhibitory concentration (MIC) of antibiotics aplicable to bifidobacteria and non-enterococcal lactic acid bacteria (LAB) 2010) for concentrations up to 1024 mg/L. The growth of all the four tested strains was not inhibited by the presence of fosfomycin.

Similarly, the tolerance of the strains to norfloxacin, a first generation synthetic fluoroquinolone occasionally used to treat common as well as complicated UTI, was studied following the same methodology. The growth of all the strains was not inhibited in concentrations as high as 1000 mg/L.

### EXAMPLE 6. Compatibility with active ingredients used for treating UTI

The compatibility of the strains of the invention with active ingredients commonly used for formulating products for treating UTI such as cranberry and vitamin C was studied *in vitro.*

Dilutions contaning 3 mg/mL of cranberry extract (tritated in proanthocyanidin ≥50 % according to European Pharmacopea) or 1 mg/mL of vitamin C were prepared in phosphate buffered saline (PBS, pH=7.4) and sterilized by filtration through 0.22 µl. One milliliter of this solutions were added to 8 mL of sterile Man, Rogosa and Sharpe (MRS) broth medium. The same amount of PBS without active ingredients was used as a control. Then, one milliliter of overnight-grown cultures of the strains of the invention (standarized to a concentration equivalent to a McFarland value of 1) were also added to the medium. Strains were then incubated for 24h at 37 °C at 5 % CO₂. The number of viable cells in the media were determined at time 0h and after 24h by plate counting in MRS solid medium.

As can be seen in FIG. 8 all the strains were able to growth in the same range regardless of the presence of the active ingredients, cranberry extract and C vitamin C which did not inhibit the growth of any of strains CECT8675, CECT8676, CECT8677 and CECT8678, among which, strains CECT8675 and CECT8677 were the ones which showed the highest capacity to grow.

### EXAMPLE 7. Formation of aggregates

Auto-aggregation capacity is one of the initial steps leading to the formation of protective biofilms against pathogens. The ability to form aggregates was evaluated by monitoring the diminution of the optical density at 620 nm of overnight cultures due to formation of aggregates and precipitation. Percent aggregation capacity (%AC) value was obtained using the following formula: %AC= (1-(OD_{tf}/ODₜ₀)/100, where OD_{tf} and ODₜ₀ are the optical density at final and initial times, respectively. OD at initial times was adjusted so that all cultures contained an equivalent number of cfu/ml. Combined cultures were prepared by mixing an appropriate amount of single strains as to obtain the desired total cfu/ml containing 50 % of each strain.

Results, as shown in Table 4 below, revealed a good aggregation capacity for strains CECT8675, CECT8676 and CECT8677, which were approximately 2-fold higher than that of VSL#3 and *L. plantarum* P17630 strains.

**TABLE 4. Aggregation capacity (%)**

| Strain | Aggregation capacity (%) |
|---|---|
| CECT8675 | 39.5 |
| CECT8676 | 40.5 |
| CECT8677 | 42.7 |
| CECT8678 | 12.0 |
| VSL#3 | 20.3 |
| P17630 | 17.3 |

### EXAMPLE 8. Production of Short Chain Fatty Acids (SCFA)

Production of SCFA was assayed by incubating the strains in basal medium supplement with fibers each one (inulin, pectin and fructo-oligosaccharides) in a specific amount, under microaerophilic conditions (5 % CO₂) at 37 °C. The amount of SCFA was determined by High Proficiency Liquid Chromatography (HPLC) as previously described [BOSCH, M. et al. Lactobacillus plantarum CECT7527, 7528 and 7529: probiotic candidates to reduce cholesterol levels. J. Sci. Food Agric. 2014. Vol 94, No. 15, pages 803-809].

All the strains were able to produce butyrate. The highest production was observed for strains CECT8677 and CECT8678, followed by the strains CECT8676 and CECT8675. All the strains were better butyrate producers than *L. plantarum* P17630.

### EXAMPLE 9. Capacity to survive to gastrointestinal and vaginal conditions

In order to evaluate the resistance of CECT8675, CECT8676, CECT8677 and CECT8678 strains to the gastrointestinal tract (GIT), assays were performed in conditions mimicking the GIT environment of mammals. Thus, survival after treatment with lysozyme, hydrogen peroxide, acidic environment and bile salts was quantified.

Twenty millilitres of overnight-grown probiotic cultures of each strain were placed in a 96-well plate which contained 200 µL of: MRS medium supplemented with 300 µg/ml lysozyme (Lysozyme Condition in Table 5), MRS supplemented with 30 µg/ml H₂O₂ (Hydrogen peroxide Condition in Table 4), MRS supplemented with 0.3 % (w/v) bile salts (SIGMA B8756-10G, 096K1213) (Hydrogen peroxide Condition in Table 5) or MRS medium adjusted to pH 2 (Acidic environment in Table 5). Survival ratio was determined for each strain by measuring the increase of optical density at 620 nm in an ELISA reader after an incubation time between 0 and 6 hours, and expressed as percentage of growth compared to that of the corresponding strain in non-supplemented MRS (used as positive control). Results are shown in Table 5 below.

**TABLE 5. Survival ratio to GIT conditions (%)**

| Condition | | CECT8675 | CECT8676 | CECT8677 | CECT8678 |
|---|---|---|---|---|---|
| Lysozyme | Oral cavity | ≥100 | ≥100 | ≥100 | ≥100 |
| Hydrogen peroxide | | ≥100 | ≥100 | ≥100 | 99 |
| Acidic environment | Stomach | ≥100 | ≥50 | ≥80 | ≥50 |
| Bile salts | Intestine | ≥100 | ≥100 | ≥100 | ≥100 |

All the strains were able to grow under the different GIT conditions assayed.

In addition, in order to evaluate the resistance of CECT8675, CECT8676, CECT8677 and CECT8678 strains to the vaginal environment, assays were performed wherein said strains were incubated in medium simulating the vaginal fluid. The vaginal fluid simulant used was that disclosed by Owen *et al.* [OWEN, D.H. et al. A vaginal fluid simulant. Contraception. 1999. Vol. 59, No. 2, pages 91-95] to which lysozyme (4-16 mg/L) was added. The final composition of said vaginal fluid simulant being 3.5 g/l NaCl, 1.4 g/l KOH, 0.22 g/l Ca(OH)₂, 0.02 g/l BSA, 2 g/l lactic acid, 1 g/l acetic acid, 0.16 g/l glycerol, 0.4 g/l urea and 5 g/l glucose; pH was further adjusted to 4.2 using lactic acid. Strains were incubated at 37 °C and 5 %CO₂.

All the CECT8675, CECT8676, CECT8677 and CECT8678 strains were also able to grow in vaginal fluid. Therefore, it was confirmed that all the strains can overcome the GIT and urogenital tract and thus exert their beneficial effect in the desired locations of the body.

### EXAMPLE 10. Combined effect of fosfomycin and the probiotic strains

The objective was to study the capacity of the probiotic strains of the present invention to increase the efficacy of fosfomycin for inhibiting pathogens associated with UTI. The activity against *Staphylococcus saprophyticus* was determined. Overnight plate cultures of *S. saprophyticus* CECT235 and of the probiotic strain *L. plantarum* CECT8677 were prepared in Tryptone Soya Agar (TSA) media and Man, Rogosa and Sharpe (MRS) media, respectively. After overnight growth of both bacteria, suspensions of *S. saprophyticus* and CECT8677 standardized to 3E+06 CFU/mL in 0.1M phosphate buffer saline (PBS) were prepared.

Then, tubes according to the following experimental conditions were prepared:
1) Positive control: Tube containing 7 mL of IST medium + 2 mL of PBS + 1 mL of the *S. saprophyticus* suspension previously prepared.
2) Antibiotic treatment: Tube containing 7 mL of IST medium + 1 mL of PBS + 1 mL of fosfomycin (1 mg/L final concentration in the tube) + 1 mL of the S. *saprophyticus* suspension previously prepared.
3) Combined treatment: Tube containing 7 mL of IST medium + 1 mL of fosfomycin (1 mg/L final concentration in the tube) + 1 mL of probiotic suspension (CECT8677) previously prepared + 1 mL of the *S. saprophyticus* suspension previously prepared.

IST medium consisted in 11 g of hydrolyzed casein, 3 g of peptone, 2 g of glucose, 3 g of sodium chloride, 1 g of soluble starch, 2 g of disodium hydrogenphosphate, 1 g of sodium acetate, 0.2 g of magnesium, glycerophosphate, 0.1 g of calcium gluconate, 0.001 g of cobalt (II) sulphate, 0.001 of copper (II) sulphate, 0.001 g of zing sulphate, 0.001 g of iron (II) sulphate, 0.002 g manganese (II) chloride, 0.001 g of menadione, 0.001 g cyanocobalamin, 0.02 g of L-cysteine hydrochloride, 0.02 g of L-tryptophan, 0.003 g of pyrodoxine, 0.003 g of pantothenate, 0.003 g of nicotinamide, 0.0003 g of biotin, 0.00004 g of thiamine, 0.01 g of adenine, 0.01 g of guanine, 0.01 g xanthine, 0.01 g of uracil and water up to 1 L.

Tubes were incubated at 37 °C in 5 % CO₂ and aliquots of 1 mL taken at times 10h and 24h for plate counting. One aliquot was used for plate counting in IST-agar medium in order to count the total number of bacteria present in the tube. Other aliquot was used for plate counting in MRS-agar containing 16 µg/mL of vancomycin for specifically counting probiotic bacteria. In tubes containing both *S. saprophyticus* and CECT8677, the concentration of *S. saprophyticus* was determined by subtracting the number of probiotic bacteria from the total count of bacteria in IST medium.

Results are presented in FIG. 9. As shown therein, the presence of fosfomycin was not enough to significantly reduce the concentration of *S. saprophyticus.* However, when probiotic was also added the presence of *S. saprophyticus* was drastically reduced. Therefore, the combined use of antibiotic and probiotic can be considered an effective management option for treating *S. saprophyticus* infections.

### EXAMPLE 11. Formulation of the product in capsules

Capsules were formulated by combining 100 mg of freeze-dried probiotic strains CECT8675 and CECT8677 (1-2E+09 cfu per capsule), 240 mg of cranberry extract, 40 mg of vitamin C (ascorbic acid), 70 mg of maltodextrin. Capsules were of hydroxypropyl methylcellulose (in this description referred as binder) and titanium dioxide (E7171) (pigment).

### EXAMPLE 12. Formulation of the product in sachets

Sachets were formulated by combining 100 mg of freeze-dried probiotic strains CECT8675 and CECT8677 (1-2E+09 cfu per sachet), 50 mg of cranberry extract, 40 mg of vitamin C (ascorbic acid) and 70 mg of maltodextrin (in this description referred as filler).

### EXAMPLE 13. Formulation of the product in vials

Probiotic product combines the ingredients present in a delivery cap and a vial. The delivery caps contained 100 mg of freeze-dried probiotic strains CECT8675 and CECT8677 (1-2 E+09 cfu per delivery cap), magnesium stearate (2.2 mg) (in this description referred as e.g. anticaking agent and antiadherent) and silicon dioxide (0.5 mg) (referred in this description as anticaking agent). The vial contains 10 mL of water and 250 mg of cranberry extract, 0.2 mg of sorbic acid (preservative), strawberry flavor (0.2 mg), citric acid (0.05 mg) and sucralose 3 mg (sweetener).

### EXAMPLE 14. Longitudinal study of the antagonistic activity of the strains

The antagonistic activity of lactobacilli strain was also studied in liquid media to have detailed information of their activity against the most comon UTI pathogens over time. Tubes containing 7 mL of ISO-sensitest liquid medium (IST) supplemented with 10 g/L of glucose were inoculated with 1 mL of PBS containing 1E+05 CFU/mL of pathogen. The pathogen strains used in this assay were *S. saprophyticus* CECT235, *E. coli* CECT5350 and *P. mirabilis* CECT434.

Subsequently, tubes were inoculated with either 1 mL of PBS contaning 1E+07 CFU/mL of probiotic. Tubes inoculated with the same amount of PBS, but not containing probiotic were used as control. Lactobacilli strains studied were *L. plantarum* CECT 8675 and CECT 8677 and *L. rhamnosus* GR-1. The antagonistic activity of the lactobacilli strains against *S. saprophyticus* is depicted in FIG. 10A. Results demonstrate that the addition of the lactobacilli to the medium reduced the amount *S. saprophyticus* live cells compared to the control medium not containing probiotic. Among the different probiotic strains tested, both *L. plantarum* CECT 8675 and CECT 8677 showed the highest antimicrobial activity, being the most efficient bacteria reducing the concentration of *S. saprophyticus* after 48h. Remarkably, *L. plantarum* CECT8677 was able to reduce the concentration of pathogen even during the first 24h, whereas *L. plantarum* CECT8677 was able to erradicate the presence of *S. saprophyticus* in the medium after 48h.

All the lactobacilli tested were also able to reduce the growth of *E. coli* (FIG. 10B). In this case all the strains showed a bacteriostatic effect during the first 24h and a slighty bactericidal activity after this period. *L. plantarum* CECT8677 was the strain showing the highest antimicrobial activity.

The antagonistic activity of the lactobacilli strains against *P. mirabillis* is depicted in FIG. 10C. *L. plantarum* CECT8675 and CECT8677 were the strains showing the highest activity during the first 24h. After this period *L. plantarum* CECT8677 was the strains with the greatest antimicrobial effect being able to reduce the presence of *P. mirabilli* to concentration even lower to those at the beginning of the assay.

In summary, results confirm that both *L. plantarum* CECT8675 and CECT8677 show good activity against UTI pathogens, being therefore good candidates for treating associated conditions.

### EXAMPLE 15. Fermentation and post-treatment of the strains

Post-treat biomass used as raw material was obtained by a manufacturing process that included the steps of fermenting and freeze-drying. A mother culture was prepared by inoculating 80 mL of MRS broth medium with the *L. plantarum* strains. The inoculated medium was fermented at 37°C in microaerophilic conditions for 48h. The resulting culture was used for inoculating 600 litres of MRS broth medium in a ratio of about 1-2% of inoculum per total volum of broth medium. The resulting medium was cooled at 3-5°C and centrifuged in order to eliminate most of the water present in the broth. Cryoprotectants were added to the concentrated culture and the resulting mixture freeze-dried at -45°C.

### BIBLIOGRAPHIC REFERENCES

ARAYA, M., et al. Guidelines for the Evaluation of Probiotics in Food - Joint FAO/WHO Working Group, FAO/WHO, Editor 2002, Food and Agriculture Organization of the United Nations and World Health Organization: Ontario, Canada.
REID, G. et al. Examination of strains of lactobacilli for properties which may influence bacterial interference in the urinary tract. J. Urol. 1987, Vol. 138, pages 330-335.
HOOTON, T.M. Clinical practice. Uncomplicated urinary tract infection. N. Engl. J. Med. 2012, Vol. 366, No. 11, pages 1028-1037.
GRABE, M. et al. European Association of Urology (EAU). Guidelines on urological infections. EAU. 2013.
SALVATORE, S. et al. Urinary Tract Infections in Women. Eur. J. Obstet. Gynecol. Reprod. Biol. 2011, Vol. 156, pages 131-136.
FOXMAN, B. and BROWN, P. Epidemiology of urinary tract infections: transmission and risk factors, incidence, and costs. Infect. Dis. Clin. North. Am. 2003, Vol. 17, No. 2, pages 227-241.
HOOTON, T.M. et al. A prospective study of risk factors for symptomatic urinary tract infection in young women. N. Engl. J. Med. 1996, Vol. 335, No. 7, pages 468-474.
JACKSON, S.L. et al. Predictors of urinary tract infection after menopause: a prospective study. Am. J. Med. 2004, Vol. 117, No. 12, pages 903-11.
ULLERYD, P. et al. Febrile urinary tract infection in men. Int. J. Antimicrob. Agents. 2003, Vol. 22, Suppl. 2, pages 89-93.
GUPTA, K. et al. International clinical practice guidelines for the treatment of acute uncomplicated cystitis and pyelonephritis in women: A 2010 update by the Infectious Diseases Society of America and the European Society for Microbiology and Infectious Diseases. Clinical Infectious Diseases. 2011, Vol. 52, No. 1, e103-e120.
ERIKSSON, A. et al. The relative importance of Staphylococcus Saprophyticus as a urinary tract pathogen: distribution of bacteria among urinary samples analyzed during 1 year at a major Swedish laboratory. APMIS. 2012, Vol. 121, pages 72-78.
GARCÍA-GARCÍA, M.I. et al. In vitro susceptibility of community-acquired urinary tract pathogens to commonly used antimicrobial agents in Spain: a comparative multicenter study (2002-2004). J. Chemotherapy. 2007, Vol. 19, pages 263-270.
ANDREU, A., et al. Etiología y sensibilidad a los antimicrobianos de los uropatógenos causantes de la infección urinaria baja adquirida en la comunidad. Estudio nacional multicéntrico. Enferm. Infecc. Microbiol. Clin. 2005, Vol. 23, No. 1, pages 4-9.
KAHLMETER, G. An international survey of the antimicrobial susceptibility of pathogens from uncomplicated urinary tract infections: the ECO·SENS Project. Journal of Antimicrobial Chemotherapy. 2003, Vol. 51, pages 69-76.
HUMMERS-PRADIER, E. et al. Urinary tract infections in adult general practice patients. British Journal of general practice. 2002, Vol. 52, pages 752-761.
SENGUPTA, K. et al. A Randomized, double blind, controlled, dose dependent clinical trial to evaluate the efficacy of a proanthocyanidin standardized whole cranberry (Vaccinium macrocarpon) powder on infections of the urinary tract. Current Bioactive Compounds. 2011, Vol. 7, No 1, pages 39-42.
BARBOSA-CESNIK, C. et al. Cranberry juice fails to prevent recurrent urinary tract infection: results from a randomized placebo-controlled trial. Clinical Infectious Diseases. 2011, Vol. 52, No. 1, pages 23-30.
KONTIOKARI, T. et al. Randomized trial of cranberry-lingonberry juice and Lactobacillus G drink for the prevention of urinary tract infections in women. BMJ. 2001. Vol. 322, pages 1-5.
JEPSON, R.G. and CRAIG, J.C. Cranberries for preventing urinary tract infections (Review). Cochrane Database Syst. Rev. 2008. Vol. 23, No. 1.
LARSSON, P.G. et al. Extended antimicrobial treatment of bacterial vaginosis combined with human lactobacilli to find the best treatment and minimize the risk of relapses. BMC Ingectious Diseases. 2011. Vol. 11, page 223
BAERHEIM, A. et al. Vaginal application of lactobacilli in the prophylaxis of recurrent lower urinary tract infection in women. Scand. J. Prim. Health Care. 1994. Vol. 12, pages 239-243.
MARTINEZ, R.C.R. et al. Improved cure of bacterial vaginosis with single dose of tinidazole (2 g), Lactobacillus rhamnosus GR-1, and Lactobacillus reuteri RC-14: a randomized, double blind, placebo control trial. Canadian Journal of Microbiology. 2009, Vol. 55, pages 133-138.
ANUKAM, K. et al. Augmentation of antimicrobial metronidazole therapy of bacterial vaginosis with oral probiotic Lactobacillus rhamnosus GR-1 and Lactobacillus reuteri RC-14: randomized, double-blind, placebo controlled trial. Microbes and infection. 2006. Vol. 8. pages 1450-1454.
BEEREPOT, M.A. et al. Lactobacilli vs antibiotics to prevent urinary tract infections: a randomized, double-blind, noninferiority trial in post-menopausal women. Archives of Internal Medicine. May 2012, Vol. 172, No. 9, pages 704-712.
EP 1137423 B1 (UREX BIOTECH, INC.) 04.10.2001, claim 4.
UEHARA, S. et al. A pilot study evaluating the safety and effectiveness of Lactobacillus vaginal suppositories in patients with recurrent urinary tract infection. International Journal of Antimicrobial Agents. 2006. Vol. 28, No. 1, pages S30-S34.
STAPLETON, A.E. et al. Randomized, placebo-controlled phase 2 trial of a Lactobacillus crispatus probiotic given intravaginally for prevention of recurrent urinary tract infection. Clin. Infect. Dis. May 2011. Vol. 52, No. 10, pages: 1212-127.
BAERHEIM, A et al. Vaginal application of lactobacilli in the prophylaxis of recurrent lower urinary tract infections in women. Scan. J. Prim Health Care, 1994. Vol. 12, pages:239-43
KOBOZIEV, I. et al. Role of the enteric microbiota in intestinal homeostasis and inflammation. Free Radical Biology & Medicine. 2014, Vol. 68, pages 122-133.
ANDREOLETTI, O. et al. The maintenance of the list of QPS microorganisms intentionally added to food or feed. Question no: EFSA-Q-2008-006. The EFSA Journal. 2008, Vol. 923, pages 1-48.
LANE, D.J. 16/23S rRNA sequencing. In Nucleic acid techniques in bacterial systematics (ed. Stackebrandt and Goodfellow). 1991, pages 177-204.
MUYZER, G. et al. Application of denaturing gradient gel electrophoresis (DGGE) and temperature gradient gel electrophoresis (TGGE) in microbial ecology. Antonie van Leeuwenhoeck. 1998. Vol 73, No 1, pages 127-141.
OLSEN, Q.J et al. Microbial Ecology and Evolution: A Ribosomal RNA Approach. Annual Review of Microbiology. 1986. Vol. 4, No. 1, pages 337- 365.
RODAS, A.M. et al. Polyphasic study of wine Lactobacillus strains: taxonomic implications. Int. J. Syst. Evol. Microbiol, 2005. Vol. 55, No. 1, pages 197-207.
NIGATU, A. et al. Randomly amplified polymorphic DNA (RAPD) for discrimination of Pediococcus pentosaceus and Pediococcus acidilactici and rapid grouping of Pediococcus isolates. Letters in Applied Microbiology. 1998. Vol 26, No. 6, pages 412-416.
JOHANSSON, M.L. et al. Randomly amplified polymorphic DNA (RAPD) for rapid typing of Lactobacillus plantarum strains. Appl. Microbiol. 1995. Vol. 21, No. 3, pages 155-159.
BOSCH, M. et al. Lactobacillus plantarum CECT7527, 7528 and 7529: probiotic candidates to reduce cholesterol levels. J. Sci. Food Agric. 2014. Vol 94, No. 15, pages 803-809.
OWEN, D.H. et al. A vaginal fluid simulant. Contraception. 1999. Vol. 59, No. 2, pages 91-95.

## Claims

1. A composition comprising an effective amount of at least a strain of *Lactobacillus plantarum,* wherein the at least strain of *Lactobacillus plantarum* is selected from the group consisting of: CECT8675 strain, CECT8676 strain, CECT8677 strain, CECT8678 strain, and a mutant of any of the deposited strains, wherein the mutant has been obtained using the deposited strain as starting material and applying mutagenesis, and wherein the obtained mutant retains or enhances at least the ability of the deposited strain to antagonize the urogenital pathogen *Staphylococcus saprophyticus.*

2. The composition according to claim 1, wherein the at least strain of *Lactobacillus plantarum* is selected from the group consisting of: CECT8675 strain, CECT8676 strain, CECT8677 strain, and CECT8678 strain.

3. The composition according to any of the claims 1-2, wherein the at least strain has been fermented in an artificial medium and submitted to a post-treatment after the fermentation, to obtain bacterial cells, and wherein the resulting bacterial cells are in a liquid medium or in a solid form.

4. The composition according to claim 3, wherein the post-treatment is selected from the group consisting of: drying, freezing, freeze-drying, fluid bed-drying, spray-drying and refrigerating in liquid medium.

5. The composition according to any of the claims 1-4, for use as a medicament or as a probiotic.

6. The composition for use according to claim 5 in the prevention and/or treatment of a condition related to alterations of the urogenital microbiota.

7. The composition for use according to claim 6 wherein the condition is urinary tract infection.

8. The composition for use according to any of the claims 5-7, in combination with an effective amount of an antimicrobial agent.

9. A pharmaceutical product, a veterinary product, a medical food, a food product, a food supplement or a personal hygiene product, comprising an effective amount of the composition as defined in any of the claims 1-8, together with appropriate amounts of acceptable excipients.

## Patentansprüche

1. Eine Zusammensetzung umfassend eine wirksame Menge von mindestens einem Stamm von *Lactobacillus plantarum,* wobei der mindestens eine Stamm von *Lactobacillus plantarum* ausgewählt ist aus der Gruppe bestehend aus: CECT8675-Stamm, CECT8676-Stamm, CECT8677-Stamm, CECT8678-Stamm, und einem Mutanten von einem der hinterlegten Stämmen, wobei der Mutant unter Verwendung von dem hinterlegten Stamm als Ausgangsmaterial und mit Anwendung von Mutagenese erhalten worden ist, und wobei der erhaltene Mutant mindestens die antagonische Fähigkeit des hinterlegten Stamms gegen das urogenitale Pathogen *Staphylococcus saprophyticus* behält oder verbessert.

2. Die Zusammensetzung nach Anspruch 1, wobei das mindestens eine Stamm von *Lactobacillus plantarum* ausgewählt ist aus der Gruppe bestehend aus: CECT8675-Stamm, CECT8676-Stamm, CECT8677-Stamm, und CECT8678-Stamm.

3. Die Zusammensetzung nach einem der Ansprüche 1-2, wobei der mindestens eine Stamm in einem künstlichen Medium fermentiert worden ist und einer Nachbehandlung nach der Fermentation unterzogen worden ist, um Bakterienzellen zu erhalten, und wobei die entstandenen Bakterienzellen in einem flüssigen Medium oder in fester Form vorhanden sind.

4. Die Zusammensetzung nach Anspruch 3, wobei die Nachbehandlung ausgewählt ist aus der Gruppe bestehend aus: Trocknung, Tiefkühlung, Gefriertrocknung, Fließbetttrocknung, Sprühtrocknung und Kühlung in einem flüssigen Medium.

5. Die Zusammensetzung nach einem der Ansprüche 1-4 zur Verwendung als Arzneimittel oder als Probiotikum.

6. Die Zusammensetzung zur Verwendung nach Anspruch 5 bei der Prävention und/oder der Behandlung von einer in Zusammenhang mit Veränderungen der urogenitalen Mikrobiota stehenden Erkrankung.

7. Die Zusammensetzung zur Verwendung nach Anspruch 6, wobei die Erkrankung eine Infektion der Harnwege ist.

8. Die Zusammensetzung zur Verwendung nach einem der Ansprüche 5-7 in Kombination mit einer wirksamen Menge von einem antimikrobiellen Mittel.

9. Ein pharmazeutisches Produkt, ein tiermedizinisches Produkt, medizinische Ernährung, ein Nahrungsmittel, ein Lebensmittelergänzungsmittel oder ein Körperpflegeprodukt, umfassend eine wirksame Menge der Zusammensetzung wie in einem der Ansprüche 1-8 definiert, zusammen mit geeigneten Mengen von annehmbaren Hilfsstoffen.

## Revendications

1. Une composition comprenant une quantité efficace d'au moins une souche de *Lactobacillus plantarum,* dans laquelle l'au moins une souche de *Lactobacillus plantarum* est choisie dans le groupe constitué de : la souche CECT8675, la souche CECT8676, la souche CECT8677, la souche CECT8678, et une mutante d'une quelconque des souches déposées, dans laquelle la mutante a été obtenue en utilisant la souche déposée comme matériau de départ et en appliquant de la mutagénèse, et dans laquelle la mutante obtenue maintien ou améliore au moins la capacité antagoniste de la souche déposée vis-à-vis le pathogène urogénital *Staphylococcus saprophyticus.*

2. La composition selon la revendication 1, dans laquelle l'au moins une souche de *Lactobacillus plantarum* est choisie dans le groupe constitué de : la souche CECT8675, la souche CECT8676, la souche CECT8677, la souche CECT8678.

3. La composition selon l'une quelconque des revendications 1-2, dans laquelle l'au moins une souche a été fermentée dans un milieu artificiel et soumise à un traitement ultérieur après la fermentation pour obtenir des cellules bactériennes, et dans laquelle les cellules bactériennes résultantes sont dans un milieu liquide ou sous forme solide.

4. La composition selon la revendication 3, dans laquelle le traitement ultérieur est choisi dans le groupe constitué de : séchage, congélation, lyophilisation, séchage à lit fluidisé, séchage par atomisation et réfrigération dans un milieu liquide.

5. La composition selon l'une quelconque des revendications 1-4, pour l'utilisation comme médicament ou comme probiotique.

6. La composition pour l'utilisation selon la revendication 5 dans la prévention et/ou le traitement d'un trouble lié à des altérations du microbiote urogénital.

7. La composition pour l'utilisation selon la revendication 6, dans laquelle le trouble est une infection des voies urinaires.

8. La composition pour l'utilisation selon l'une quelconque des revendications 5-7 en combinaison avec une quantité efficace d'un agent antimicrobien.

9. Un produit pharmaceutique, un produit vétérinaire, un aliment médical, un produit alimentaire, un supplément alimentaire ou un produit d'hygiène personnelle, comprenant une quantité efficace de la composition telle que définie dans l'une quelconque des revendications 1-8 conjointement avec des quantités appropriées d'excipients.


### SEQUENCE LISTING

<110> AB-Biotics, S.A.
<120> Probiotic strains for urinary tract infections
<130> PD10106PC00
<160> 4
<170> PatentIn version 3.5
<210> 1
   <211> 1299
   <212> DNA
   <213> Lactobacillus plantarum
<400> 1
   acttggaccg catggtccga gcttgaaaga tggcttcggc tatcactttt ggatggtccc      60
   gcggcgtatt agctagatgg tggggtaacg gctaccatgg caatgatcgt agccgacctg     120
   agagggtaat cggccacatt gggactgaga cacggcccaa actcctacgg gaggcagcag     180
   tagggaatct tccacaatgg acgaaagtct gatggagcaa cgccgcgtga gtgaagaagg     240
   gtttcggctc gtaaaactct gttgttaaag aagaacatat ctgagagtaa ctgttcaggt     300
   attgacggta tttaaccaga aagccacggc taactacgtg ccagcagccg cggtaatacg     360
   taggtggcaa gcgttgtccg gatttattgg gcgtaaagcg agcgcaggcg gttttttaag    420
   tctgatgtga aagccttcgg ctcaaccgaa gaagtgcatc ggaaactgga aaacttgagt     480
   gcagaagagg acagtggaac tccatgtgta gcggtgaaat gcgtagatat atggaagaac     540
   accagtggcg aaggcggctg tctggtctgt aactgacgct gaggctcgaa agtatggtag     600
   caaacaggat tagataccct ggtagtccat accgtaacga tgaatgctaa gtgttggagg     660
   gtttccgccc ttcagtgctg cagctaacgc attaagcatt ccgcctgggg agtacggccg     720
   caaggctgaa actcaaagga attgacgggg cccgcaccaa gcggtggagc atgtggtttt     780
   attcgaagct acgcgaagaa ccttaccagg tcttgacata ctatgcaaat ctaagagatt     840
   agacgttccc ttcgggacat ggatacaggt ggtggcatgg ttgtcggtca gctcgtgtcg     900
   tggaaatgtt gggttaagtc ccgcaacgag cgcaaccctt attatcagtt gccagcatta     960
   agttgggcac tctggtgaga actgccggtg acaaaccgga ggaaggtggg gatgacgtca    1020
   aatcatcatg ccccttatga cctgggctac acacgtgcta caatggatgg tacaacgagt    1080
   tgcgaactcg cgagagtaag ctaatctctt aaagccattc tcagttcgga ttgtaggctg    1140
   caactcgcct acatgaagtc ggaatcgcta gtaatcgcgg atcagcatgc cgcggtgaat    1200
   acgttcccgg gccttgtaca caccgcccgt cacaccatga gagtttgtaa cacccaaagt    1260
   cggtggggta acctttagaa ccagccgcct aagggggac                             1299
<210> 2
   <211> 1436
   <212> DNA
   <213> Lactobacillus plantarum
<400> 2
   gcagtcgacg actctggtat tgataggtgc ttgcatcatg atttacattt gagagagtgg 60
   cgaactggtg agtaacacgt gggaaacctg cccagagcgg gggataacac ctggaaacag 120
   atgctaatac cgcataacaa cttggaccgc atggtccgag gttgaaagat ggcttcggct 180
   atcacttttg gatggtcccg cggcgtatta gctagatggt ggggtaacgg ctcaccatgg 240
   caatgatacg tagccgacct gagagggtaa tcggccacat tgggactgag acacggccca 300
   aactcctacg ggaggcagca gtagggaatc ttccacaatg gacgaaagtc tgatggagca 360
   acgccgcgtg agtgaagaag ggtttcggct cgtaaaactc tgttgttaaa gaagaacata 420
   tctgagagta actgttcagg tattgacggt atttaaccag aaagccacgg ctaactacgt 480
   gccagcagcc gcggtaatac gtaggtggca agcgttgtcc ggatttattg ggcgtaaagc 540
   gagcgcaggc ggttttttaa gtctgatgtg aaagccttcg gctcaaccga agaagtgcat 600
   cggaaactgg gaaacttgag tgcagaagag gacagtggaa ctccatgtgt agcggtgaaa 660
   tgcgtagata tatggaagaa caccagtggc gaaggcggct gtctggtctg taactgacgc 720
   tgaggctcga aagtatgggt agcaaacagg attagatacc ctggtagtcc ataccgtaaa 780
   cgatgaatgc taagtgttgg agggtttccg cccttcagtg ctgcagctaa cgcattaagc 840
   attccgcctg gggagtacgg ccgcaaggct gaaactcaaa ggaattgacg ggggcccgca 900
   caagcggtgg agcatgtggt ttaattcgaa gctacgcgaa gaaccttacc aggtcttgac 960
   atactatgca aatctaagag attagacgtt ccctccgggg acatggatac aggtggtgca 1020
   tggttgtcgt cagctcctgt cgtgagaatg gtgggttaag tcccgcaacg agcgcaaccc 1080
   ttattatcag tggccagcat taagttgcgc actctggtga gactgccggt gacaaaccgg 1140
   aggaaggtgg ggatgacgtc aaatcatcat gccccttatg acctgggcta cacacgtgct 1200
   acaatggatg gtacaacgag ttgcgaactc gcgagagtaa gctaatctct taaagccatt 1260
   ctcagttcgg attgtaggct gcaactcgcc tacatgaagt cggaatcgct agtaatcgcg 1320
   gatcagcatg ccgcggtgaa tacgttcccg ggccttgtac acaccgcccg tcactccatg 1380
   agagtttgta acacccaaag tcggtggggt aacctttagg aaccagccgc taaagg 1436
<210> 3
   <211> 1318
   <212> DNA
   <213> Lactobacillus plantarum
<400> 3
   cataaacaac ttggaccgcc atgttccgag cttaaaaatg cttcggctat cacttttgaa 60
   tggtccccgc ggcgtattag ctagatggtg gggtaacggc tcaccatggc aatgatacgt      120
   agccgacctg agagggtaat cggccacatt gggactgaga cacggcccaa actcctacgg      180
   gaggcagcag tagggaatct tccacaatgg acgaaagtct gatgagggag caacgccgcg      240
   tgagtgaaga agggtttcgg ctcgtaaaac tctgttgtta aagaagaaca tatctgagag      300
   taactgttca ggtattgacg gtatttaacc agaaagccac ggctaactac gtgccagcag      360
   ccgcggtaat acgtaggtgg caagcgttgt ccggatttat tgggcgtaaa gcgagcgcag      420
   gcggtttttt aagtctgatg tgaaagcctt cggctcaacc gaagaagtgc atcggaaact      480
   gggaacttga gtgcagaaga ggacagtgga actccatgtg tagcggtgaa atgcgtagat      540
   atatggaaga acaccagtgg cgaaggcggc tgtctggtct gtaactgacg ctgaggctcg      600
   aaagtatggg tagcaaacag gattagatac cctggtagtc cataccgtaa acgatgaatg      660
   ctaagtgttg gagggtttcc gcccttcagt gctgcagcta acgcattaag cattccgcct      720
   gggagtacgg ccgcaaggct gaaactcaaa ggaattgacg ggggcccgca caagcggtgg      780
   agcatgtggt ttaattcgaa gctacgcgaa gaaccttacc aggtcttgac atactatgca      840
   aatctaagag attagacgtt cccttcgggg acatggatac aggtggtgca tggttgtcgt      900
   cagctcgtgt cgtgagaatg ttgggttaag tcccgcaacg agcgcaaccc tttatttatc      960
   agttgccagc attaagttgg gcactctctg gtgaagaact gccggtgaca aaccggagga     1020
   aggtggggat gacgtcaaat catcatgccc cttatgacct gggctacaca cgtgctacaa     1080
   tggatggtac aacgagttgc gaactcgcga gagtaagcta atctcttaaa gccattctca     1140
   gttcggattg taggctgcaa ctcgcctaca tgaagtcgga atcgctagta atcgcggatc     1200
   agcatgccgc ggtgaatacg ttcccgggcc ttgtacacac cgcccgtcac accatgagag     1260
   tttgtaacac ccaaagtcgg tggggtaacc tttaggaacc acccgctaag gggaccac        1318
<210> 4
   <211> 1389
   <212> DNA
   <213> Lactobacillus plantarum
<400> 4
   gggggcgaac tgtgtgagta accacgtggg aaacctgccc agaagcggga gataaacacc      60
   tggaaacaga tgcttaatac cgcataacaa cttggaccgc atggtccgag cttgaaagat     120
   ggcttcggct atcacttttg gatggtcccg cggcgtatta gctagatggt ggggtaacgg     180
   ctccaccatg gcaatgatac gtagccgacc tgagagggta atcggccaca ttgggactga     240
   gacacggccc aaactcctac gggaggcagc agtagggaat cttccacaat ggacgaaagt     300
   ctgatggagc aacgccgcgt gagtgaagaa gggtttcggc tcgtaaaact ctgttgttaa     360
   agaagaacat atctgagagt aactgttcag gtattgacgg tatttaacca gaaagccacg     420
   gctaactacg tgccagcagc cgcggtaata cgtaggtggc aagcgttgtc cggatttatt     480
   gggcgtaaag cgagcgcagg cggtttttta agtctgatgt gaaagccttc ggctcaaccg     540
   aagaagtgca tcggaaactg ggaaacttga gtgcagaaag aggacagtgg aactccatgt     600
   gtagcggtga aatgcgtaga tatatggaag aacaccagtg gcgaaggcgg ctgtctggtc     660
   tgtaactgac gctgaggctc gaaagtatgg gtagcaaaca ggattagata ccctggtagt     720
   ccataccgta aacgatgaat gctaagtgtt ggagggtttc cgcccttcag tgctgcagct     780
   aacgcataag cattccgcct ggggagtacg gccgcaaggc tgaaactcaa aggattgacg     840
   ggggcccgca caagcggtgg agcatgtggt ttaattcgaa gctacgcgaa gaaccttacc     900
   aggtcttgac atactatgca aatctaagag attagacgtt cccttcgggg acatggataa     960
   caggtggtgc atggttgtcc gtcagcctcg tgtcgtgaga tgttgggtta agtcccgcaa    1020
   cgagcgcaac ccttattata cagttgccag acataaagtt gggcactctg gtgagactgc    1080
   cggtgacaaa ccggagaaag gtggagatga cgtcaaatca tctatgcccc ttatgacctg    1140
   ggctacacac gtgctacaat ggatgtgcac aacgagtgtg cgacatctca gcgagagata    1200
   agctaatctc ttaaagccat tctcagttcg gattgtaggc tgcaactcgc ctacatgaag    1260
   tcggaatcgc tagtaatcgc ggatcagcat gccgcggtga atacgttccc gggccttgta    1320
   cacaccgccc tctcttccta gagtttgtaa cacccaaagt cggtggggta actttagaac    1380
   cagccgctt                                                            1389